# EUROPEAN PATENT APPLICATION

(11) **EP 4 379 045 A1**
(43) Date of publication of application: **05.06.2024**
(21) Application number: 22849624.6
(22) Date of filing: 29.07.2022
(51) Int. Cl.: C12N 5/079, A61K 35/30, A61P 25/00, A61P 25/02, C12N 1/00

(54) **METHOD FOR PRODUCING HIGHLY PROLIFERATIVE CELL, AND HIGHLY PROLIFERATIVE CELL AND USE THEREOF**

(30) Priority: 29.07.2021 JP 2021124172
(71) Applicant: Tokyo Medical University, Tokyo 160-8402 (JP)
(72) Inventor: OCHIYA Takahiro, Tokyo 160-8402 (JP); SHINODA Tatsuya, Tokyo 100-6606 (JP); TAKIZAWA Kazuya, Tokyo 100-6606 (JP)
(74) Representative: D Young & Co LLP
(86) International application number: PCT/JP2022/029317
(87) International publication number: WO 2023/008564

(57) **Abstract**

A production method for highly proliferating cells or ectodermal progenitor cells, the production method including (i) preparing ectodermal cells which serve as a raw material, (ii) culturing the ectodermal cells which serve as the raw material in a medium containing a small-molecule signaling pathway inhibitor by bringing the inhibitor into contact with the ectodermal cells, and (iii) after the contact, performing additional culturing in a medium containing the inhibitor to obtain a culture containing highly proliferating cells that have higher cell proliferative ability than the ectodermal cells which serve as the raw material; highly proliferating cells having characteristics of ectodermal cells and proliferative ability that allows the number of the highly proliferating cells after more than 28 days of a culturing period during which the cells are brought into contact with a small-molecule signaling pathway inhibitor to be greater than 1.0 times the number of ectodermal cells which are cultured under the same culturing conditions, except that the cells are not brought into contact with the inhibitor, for the same culturing period; a cell-secreted product derived from these highly proliferating cells or the highly proliferating cells obtained by the above production method for highly proliferating cells; a cell-secreted product including a predetermined protein and/or miRNA; a pharmaceutical composition for use in prevention or treatment of a disorder associated with peripheral nerve cells or central nerve cells, the pharmaceutical composition including these cell-secreted products; and an inhibition method for sympathetic nervous system, the inhibition method including bringing neurons into contact with a cell-secreted product secreted from the highly proliferating cells obtained by the above production method or the above highly proliferating cells.

## Description

### Technical Field

The present invention relates to a production method for highly proliferating cells and highly proliferating cells and use thereof.

### Background Art

In the field of regenerative medicine, various cells in the living body, cells at various differentiation stages, or cells that have been induced to differentiate in a specific direction are used. Considering that these cells are to be used in living bodies, it is desirable that no genetic modification takes place in these cells. Up until now, it has been reported that, by bringing mature endodermal cells into contact with a specific low-molecular compound, the mature cells can be reprogrammed into stem cells or progenitor cells without genetic modification (WO 2017/119512 A and WO 2020/080550 A).

### Summary of Invention

Meanwhile, many of the cells that are obtained without performing genetic modification, especially cells that are comparatively close to mature cells, have limited proliferative ability and tend to be incapable of being maintained in vivo and in vitro over a long period of time. For this reason, the need for highly proliferating cells that are effective in long-term usage of the function of the cells or mass production of a useful substance generated by the cells still remains. In particular, ectodermal cells, including cells of the nervous system, are not being fully utilized, unlike endodermal cells.

### Technical Problem

An object of the present disclosure is to provide a method for producing highly proliferating cells and highly proliferating cells and use thereof.

### Solution to Problem

As a result of intensive research, the present inventors found that highly proliferating cells are obtained by bringing a small-molecule signaling pathway inhibitor into contact with ectodermal cells.

Specifically, the present disclosure includes the following embodiments.
[1] A method for producing highly proliferating cells, the method including
   (i) preparing ectodermal cells which serve as a raw material,
   (ii) culturing the ectodermal cells which serve as the raw material in a medium containing a small-molecule signaling pathway inhibitor by bringing the inhibitor into contact with the ectodermal cells, and
   (iii) after the contact, performing additional culturing in a medium containing the inhibitor to obtain a culture containing highly proliferating cells that have higher cell proliferative ability than the ectodermal cells which serve as the raw material;
[2] The production method according to [1], in which the highly proliferating cells have proliferative ability that allows the number of the highly proliferating cells after more than 28 days of a culturing period during which the cells are brought into contact with the inhibitor to be greater than 1.0 times the number of ectodermal cells which are cultured under the same culturing conditions, except that the cells are not brought into contact with the inhibitor, for the same culturing period;
[3] The production method according to [1] or [2], in which the ectodermal cells which serve as the raw material contain cells of the central nervous system;
[4] The production method according to any one of [1] to [3], in which the ectodermal cells which serve as the raw material contain astrocytes;
[5] The production method according to any one of [1] to [4], in which the inhibitor contains at least one compound selected from the group consisting of a TGFβ receptor inhibitor and a ROCK inhibitor;
[6] The production method according to [5], in which the concentration of the TGFβ receptor inhibitor is in a range of 0.001 µM to 100 µM;
[7] The production method according to [5] or [6], in which the concentration of the ROCK inhibitor is in a range of 0.001 µM to 100 µM;
[8] The production method according to any one of [1] to [7], in which the highly proliferating cells express at least one gene specific to mature cells at the same level as or at a higher level than the ectodermal cells which serve as the raw material, and express at least one gene specific to progenitor cells at the same level as or at a higher level than the ectodermal cells which serve as the raw material;
[9] The production method according to any one of [1] to [8], in which the highly proliferating cells are negative for at least one selected from the group consisting of Musashi1, Notch1, Nestin, and SOX2;
[10] Highly proliferating cells having characteristics of ectodermal cells and proliferative ability that allows the number of the highly proliferating cells after more than 28 days of a culturing period during which the cells are brought into contact with a small-molecule signaling pathway inhibitor to be greater than 1.0 times the number of ectodermal cells which are cultured under the same culturing conditions, except that the cells are not brought into contact with the inhibitor, for the same culturing period;
[11] The cells according to [10], in which the highly proliferating cells contain cells negative for at least one selected from the group consisting of Musashi1, Notch1, Nestin, and SOX2;
[12] The cells according to [10] or [11], in which the NG2 expression level is higher than the NG2 expression level in the ectodermal cells that are not brought into contact with the inhibitor;
[13] A method for producing a cell-secreted product, the method including:
   obtaining a culture containing a cell-secreted product secreted from the highly proliferating cells obtaining by the production method according to any one of [1] to [9] or the highly proliferating cells according to any one of [10] to [12]; and
   separating the cell-secreted product form the culture;
[14] The method according to [13], in which the cell-secreted product is an exosome;
[15] A method for producing ectodermal progenitor cells, the method including:
   (i) preparing ectodermal cells which serve as a raw material,
   (ii) culturing, for a period of more than 28 days, the ectodermal cells which serve as the raw material in a medium containing a small-molecule signaling pathway inhibitor by bringing the inhibitor into contact with the ectodermal cells, and
   (iii) after the contact, performing additional culturing in a medium containing the inhibitor to obtain a culture containing highly proliferating cells that have higher cell proliferative ability than the ectodermal cells which serve as the raw material,
   in which the ectodermal progenitor cells are the highly proliferating cells;
[16] The production method according to [15], further including:
   isolating the highly proliferating cells from the culture;
[17] A cell-secreted product containing:
   a protein and/or a miRNA,
   in which the protein contains a combination of vinculin (P18206), integrin β-1, CD29 (P05556), pyruvate kinase M1/2 (P14618), and ephrin type-A receptor 2 (P29317), and
   in which the miRNA contains a combination of hsa-miR-382-5p (MIMAT0000737), hsa-miR-155-5p (MIMAT0000646), hsa-miR-379-5p (MIMAT0000733), hsa-miR-16-5p (MIMAT0000069), hsa-miR-382-5p (MIMAT0000737), hsa-miR-16-5p (MIMAT0000069), hsa-miR-382-5p (MIMAT0000737), hsa-miR-21-5p (MIMAT0000076), hsa-let-7a-5p (MIMAT0000062), hsa-miR-16-5p (MIMAT0000069), hsa-miR-409-3p (MIMAT0001639), hsa-let-7a-5p (MIMAT0000062), and hsa-let-7f-5p (MIMAT0000067) ;
[18] The cell-secreted product according to [17], which is an exosome;
[19] A pharmaceutical composition for use in prevention or treatment of a disorder associated with peripheral nerve cells or central nerve cells, the pharmaceutical composition containing any one of:
   a cell-secreted product secreted from the highly proliferating cells obtained by the production method according to [1] or [2] or from the highly proliferating cells according to any one of [10] to [12], and
   the cell-secreted product according to [17] or [18]; and
[20] A method for inhibiting sympathetic nervous system, the method including:
   bringing neurons into contact with any one of:
   a cell-secreted product secreted from the highly proliferating cells obtained by the production method according to [1] or [2] or from the highly proliferating cells according to any one of [10] to [12], and
   the cell-secreted product according to [17] or [18].

### Effects of Invention

According to the present disclosure, a method for producing highly proliferating cells and highly proliferating cells and use thereof can be provided.

### Brief Description of Drawings

Fig. 1 shows the forms of ectodermal cells after long-term culturing according to Example 1.
Fig. 2 shows the forms of ectodermal cells before and after replacing the medium to a collection medium according to Example 2.
Fig. 3A shows the particle size distribution of particles in a culture supernatant collected after additional culturing of the normal group according to Example 2.
Fig. 3B shows the particle size distribution of particles in a culture supernatant collected after additional culturing of the NHA-YA group according to Example 2.
Fig. 4A shows proteins selected by performing proteome analysis on NHA-YA-derived exosomes according to Example 4.
Fig. 4B shows proteins selected by performing proteome analysis on NHA-YA-derived exosomes according to Example 4.
Fig. 5 shows miRNAs as Parkinson's disease markers which are selected by performing miRNA analysis on NHA-YA-derived exosomes according to Example 5.
Fig. 6 shows miRNAs as Alzheimer's disease markers which are selected by performing miRNA analysis on NHA-YA-derived exosomes according to Example 5.
Fig. 7 shows miRNAs as Alzheimer's disease markers which are selected by performing miRNA analysis on NHA-YA-derived exosomes according to Example 5.
Fig. 8 shows miRNAs as Alzheimer's disease markers which are selected by performing miRNA analysis on NHA-YA-derived exosomes according to Example 5.
Fig. 9 shows miRNAs as Alzheimer's disease markers which are selected by performing miRNA analysis on NHA-YA-derived exosomes according to Example 5.
Fig. 10 shows miRNAs as depression markers which are selected by performing miRNA analysis on NHA-YA-derived exosomes according to Example 5.

### Description of Embodiments

### <1> Production Method for Highly Proliferating Cells

The production method for highly proliferating cells according to the present disclosure includes:
(i) preparing ectodermal cells which serve as a raw material,
(ii) culturing the ectodermal cells which serve as the raw material in a medium containing a small-molecule signaling pathway inhibitor by bringing the inhibitor into contact with the ectodermal cells, and
(iii) after the contact, performing additional culturing in a medium containing the inhibitor to obtain a culture containing highly proliferating cells that have higher cell proliferative ability than the ectodermal cells which serve as the raw material.

In the present specification, the term "cell" should be interpreted as including at least one cell, unless otherwise specified. Therefore, unless otherwise specified, the term "cell" is not limited to a single cell, and can be used synonymously with a cell population.

In the present specification, the term "cell population" should be interpreted as including at least one type of cells, unless otherwise specified. Therefore, unless otherwise specified, the term "cell population" is not limited to one type of cells.

"Ectoderm" is one of the three germ layers that arise during the development of metazoans. The ectoderm originates from the outer layer of the embryo and develops into skin, nervous system, and sensory organs. Examples of the skin include epidermis, hair, nails, and cutaneous gland; examples of the nervous system include cranial nerve, spinal cord, and peripheral nerve; and examples of the sensory organs include a visual organ, an auditory organ, a balance organ, a gustatory organ, an olfactory organ, and a tactile organ.

The "ectodermal cells" used in the present disclosure include, for example, cells of the nervous system such as cells of the central nervous system and cells of the peripheral nervous system. Therefore, in the present disclosure, the "ectodermal cells which serve as raw material" can include, for example, cells of the central nervous system. The ectodermal cells which serve as the raw material may include astrocytes.

The ectodermal cells used in the production method of the present disclosure may be provided from any source, such as a mammal. Examples of the mammal include a human, a rat, a mouse, a cat, a dog, a guinea pig, a rabbit, a sheep, a horse, a pig, a cow, and a monkey. The mammal is preferably a human, a rat, a mouse, a cat, or a dog and more preferably a human, a rat, or a mouse.

The "ectodermal cells" used in the present disclosure are cells constituting "ectodermal tissues" and "ectodermal organs". Examples of the "ectodermal tissues" and "ectodermal organs" can include central nerve (brain and spinal cord), pituitary gland, peripheral nerve, enteric nerve, adrenal medulla, melanocytes, facial cartilage, tooth dentine, epidermis, hair, nails, skin, sebaceous gland, salivary gland, sweat gland, mammary gland, nasal cavity, nasal mucosa, oral epithelium, oral cavity, eyes, bladder, and anus. The brain can be broadly divided into cerebrum, cerebellum, and brainstem. The cerebrum is further divided into telencephalon and diencephalon, and the brainstem is further divided into mesencephalon, pons, and medulla oblongata. The central nerve is composed of neurons and glial cells, and these cells can be chosen as the starting material. These tissues or organs contain many mature cells that have completed the final differentiation stage.

As the ectodermal cells which serve as the raw material, for example, at least one selected from the group consisting of astrocytes, microglia, and oligodendrocytes, classified as glial cells, oligodendrocyte progenitor cells (also referred to as polydendrocytes), ependymal cells, Schwann cells, and satellite cells can be selected. These cells may be ectodermal cells induced from primary cultured cells obtained from a living body, an established cell line, or pluripotent stem cells such as ES cells or induced pluripotent stem cells (iPS cells).

The cells used in the method of the present disclosure may be, for example, cells isolated and purified from the brain extracted from a mammal.

For example, in the case of rats, although it is preferable to use the brains extracted from adult rats aged 10 weeks to 20 weeks old, the brains from young rats aged 2 months or younger may also be used. In the case of humans, the cells can be obtained by biopsy or surgery. For example, both neurons and glial cells can be collected by biopsy. In the case of surgery, an excised brain tissue fragment of an adult or the brain excised from a dead fetus can be used. Alternatively, it is also possible to use neurons or glial cells that have been frozen after being isolated and purified from these extracted brains.

The ectodermal cells which serve as the raw material can be defined as cells exhibiting the characteristics of ectodermal cells. Examples of the characteristics of ectodermal cells can include the form of the cells, the expression of a gene specific to the cells (may also be referred to as a "marker gene"), and the like. Ectodermal cells can be identified, for example, by the expression of at least one marker gene selected from the group consisting of GFAP, S100B, SLC1A2 (also known as "EAAT2" or "GLT-1"), and NG2 in any differentiation stages. The expression of the marker genes of ectodermal cells may be confirmed based on genes or proteins. The confirmation of the expression of the genes or proteins can be performed by a method known in the art, such as the presence or absence of the expression, the specific expression level based on a measurement method, or comparison of the expression level with that of the same gene or protein in specific cells.

Here, in the present specification, the expression of a marker gene can be confirmed using a technique known in the art, for example, by measuring the expression level of a gene using quantitative PCR (may be described as "qPCR") or the like, or by measuring the protein abundance using immunoassay such as ELISA or flow cytometry.

The ectodermal cells which serve as the raw material that are prepared as described above are brought into contact with a small-molecule signaling pathway inhibitor. The contact may be performed in vitro. The small-molecule signaling pathway inhibitor that can be used in the present method is not particularly limited, and any small-molecule signaling pathway inhibitor can be used. Examples of the small-molecule signaling pathway inhibitor include a transforming growth factor (TGF) β receptor inhibitor, a Rho-associated protein kinase (ROCK) inhibitor, and a glycogen synthase kinase 3 (GSK3) inhibitor.

In the production method according to the present disclosure, the inhibitor may contain at least one compound selected from the group consisting of a TGFβ receptor inhibitor, a ROCK inhibitor, and a GSK3 inhibitor. In the production method according to the present disclosure, the inhibitor may contain at least one compound selected from the group consisting of a TGFβ receptor inhibitor and a ROCK inhibitor.

The TGFβ receptor inhibitor is not particularly limited as long as it has the action of inhibiting the function of the TGFβ receptor, and examples thereof include 2-(5-benzo[1,3]dioxol-4-yl-2-tert-butyl-1H-imidazol-4-yl)-6-methylpyridine, 3-(6-methylpyridin-2-yl)-4-(4-quinolyl)-1-phenylthiocarbamoyl-1H-pyrazole (A-83-01), [2-(5-choloro-2-fluorophenyl)-4-(4-pyridylamino)]pteridine (SD-208), 3-(pyridin-2-yl)-4-(4-quinonyl)]-1H-pyrazole, and 2-(3-(6-methylpyridin-2-yl)-1H-pyrazol-4-yl)-1,5-naphthyridine (all of the above manufactured by Merck KGaA), SB431542 (Sigma-Aldrich, Inc.), and CultureSure (registered trademark) A-83-01 (FUJIFILM Wako Pure Chemical Corporation). The TGFβ receptor inhibitor is preferably CultureSure (registered trademark) A-83-01. The TGFβ receptor inhibitors also include a TGFβ receptor antagonist. Only one type of the TGFβ receptor inhibitor may be used, or two or more types thereof may be used in combination.

The ROCK inhibitor is not particularly limited as long as it has the action of inhibiting the function of a Rho-associated protein kinase, and examples of the ROCK inhibitor include GSK269962A (Axon Medchem BV), Fasudil hydrochloride (Tocris Bioscience), CultureSure (registered trademark) Y-27632 (FUJIFILM Wako Pure Chemical Corporation), and H-1152 dihydrochloride (FUJIFILM Wako Pure Chemical Corporation). The ROCK inhibitor is preferably CultureSure (registered trademark) Y-27632. Only one type of the ROCK inhibitor may be used, or two or more types thereof may be used in combination.

The GSK3 inhibitor is not particularly limited as long as it has the action of inhibiting the function of glycogen synthase kinase (GSK) 3, and examples of the GSK3 inhibitor include SB216763 (Selleck Chemicals LLC), CHIR 98014 (Axon Medchem BV), CHIR 99021 (Axon Medchem BV), SB415286 (Tocris Bioscience), and Kenpaullone (Cosmo Bio Co., LTD.). The GSK3 inhibitor is preferably CHIR 99021. Only one type of the GSK3 inhibitor may be used, or two or more types thereof may be used in combination.

The small-molecule signaling pathway inhibitor used in the present production method can be at least one selected from the group consisting of a TGFβ receptor inhibitor and a ROCK inhibitor. That is, the small-molecule signaling pathway inhibitor used in the present production method may be:
·a combination of a TGFβ receptor inhibitor and a ROCK inhibitor,
·a TGFβ receptor inhibitor, or
·a ROCK inhibitor.
In one aspect, the small-molecule signaling pathway inhibitor that is used is a combination of a TGFβ receptor inhibitor and a ROCK inhibitor. In the present specification, the "small-molecule signaling pathway inhibitor" may be simply referred to as an "inhibitor".

As an aspect of the present disclosure, for example, the following inhibitors are included:
(1) at least one compound selected from the group consisting of 2-(5-benzo[1,3]dioxol-4-yl-2-tert-butyl-1H-imidazol-4-yl)-6-methylpyridine, 3-(6-methylpyridin-2-yl)-4-(4-quinolyl)-1-phenylthiocarbamoyl-1H-pyrazole (A-83-01), [2-(5-chloro-2-fluorophenyl)-4-(4-pyridylamino)]pteridine (SD-208), 3-(pyridin-2-yl)-4-(4-quinonyl)]-1H-pyrazole, 2-(3-(6-methylpyridin-2-yl)-1H-pyrazol-4-yl)-1,5-naphthyridine, SB431542, and CultureSure (registered trademark) A-83-01;
(2) a combination of:
   at least one compound selected from the group consisting of 2-(5-benzo[1,3]dioxol-4-yl-2-tert-butyl-1H-imidazol-4-yl)-6-methylpyridine, 3-(6-methylpyridin-2-yl)-4-(4-quinolyl)-1-phenylthiocarbamoyl-1H-pyrazole (A-83-01), [2-(5-chloro-2-fluorophenyl)-4-(4-pyridylamino)]pteridine (SD-208), 3-(pyridin-2-yl)-4-(4-quinonyl)]-1H-pyrazole, 2-(3-(6-methylpyridin-2-yl)-1H-pyrazol-4-yl)-1,5-naphthyridine, SB431542, and CultureSure (registered trademark) A-83-01, and
   at least one compound selected from the group consisting of GSK269962A, Fasudil hydrochloride, CultureSure (registered trademark) Y-27632, and H-1152 dihydrochloride; and
(3) at least one compound selected from the group consisting of GSK269962A, Fasudil hydrochloride, CultureSure (registered trademark) Y-27632, and H-1152 dihydrochloride.

The concentration of the TGFβ receptor inhibitor in the medium is, for example, in a range of 0.001 µM to 100 µM, 0.01 µM to 50 µM, or 0.05 µM to 30 µM, and the concentration can be suitably adjusted in accordance with the type of the TGFβ receptor to be used. In one aspect, the concentration of the TGFβ receptor inhibitor, for example, CultureSure (registered trademark) A-83-01, in the medium is 0.1 µM to 3 µM.

The concentration of the ROCK inhibitor in the medium is, for example, in a range of 0.001 µM to 100 µM, 0.01 µM to 80 µM, or 0.1 µM to 50 µM, and the concentration can be suitably adjusted in accordance with the type of the ROCK inhibitor to be used. In one aspect, the concentration of the ROCK inhibitor, for example, CultureSure (registered trademark) Y-27632, in the medium is 1 µM to 30 µM.

The concentration of the GSK3 inhibitor in the medium is, for example, in a range of 0.001 µM to 100 µM, 0.01 µM to 80 µM, or 0.1 µM to 50 µM, and the concentration can be suitably adjusted in accordance with the type of the GSK3 inhibitor to be used.

The above concentration ranges can be applied to both a case where the TGFβ receptor inhibitor, the ROCK inhibitor, and the GSK3 inhibitor are each used solely and a case where these inhibitors are used in combination. Furthermore, in a case where the inhibitors are insoluble or sparingly soluble in water, the inhibitors can be dissolved in a small amount of an organic solvent with low toxicity (for example, DMSO or the like) and then added to the medium to the final concentration described above.

The contact between the ectodermal cells which serve as the raw material and the small-molecule signaling pathway inhibitor is performed by culturing the ectodermal cells which serve as the raw material in the medium containing the inhibitor. Specifically, the culturing is performed by adding the inhibitor to the medium at an effective concentration.

After bringing the ectodermal cells which serve as the raw material into contact with the small-molecule signaling pathway inhibitor, the ectodermal cells are subjected to further culturing in a medium containing the inhibitor. Here, further culturing is also referred to as "additional culturing" or "second culturing". On the other hand, culturing until the point at which the ectodermal cells which serve as the raw material are brought into contact with the small-molecule signaling pathway inhibitor, in other words, culturing of the ectodermal cells in the medium that does not contain the small-molecule signaling pathway inhibitor is also referred to as "pre-culturing" or "first culturing". By performing the additional culturing after the contact with the inhibitor, highly proliferating cells with increased cell proliferative ability are obtained. Here, since the medium in the additional culturing also contains the inhibitor, the contact between the cells and the inhibitors is continued.

In the present disclosure, as the media used for the pre-culturing and the additional culturing, a medium used for culturing a wide range of animal cells can be used as a basal medium, and a commercially available basal medium may be used. Examples of the commercially available basal medium include an astrocyte medium (Astrocyte Growth Medium; AGM), minimum essential medium (MEM), Dulbecco's modified minimum essential medium (DMEM), RPMI1640 medium, 199 medium, Ham's F12 medium, William's E medium, and NS basal medium (FUJIFILM Wako Pure Chemical Corporation), but the basal medium is not limited thereto. The above medium may be used solely, or two or more kinds thereof may be used in combination.

Examples of an additive to be added to the medium include a cytokine, a growth factor (for example, epidermal growth factor (EGF) and fibroblast growth factor-2 (FGF-2)), a hormone (for example, insulin, estradiol, progesterone, testosterone, and thyroxine), a steroid (for example, dexamethasone (Dex)), a plasma-derived protein (for example, transferrin), various amino acids (for example, L-glutamine and L-proline), various inorganic salts (for example, selenite and NaHCO₃), various vitamins (for example, nicotinamide and an ascorbic acid derivative), N2 supplement (FUJIFILM Wako Pure Chemical Corporation), NS supplement (FUJIFILM Wako Pure Chemical Corporation), B-27 Plus Supplement (Thermo Fisher Scientific Inc.), various antibiotics (for example, penicillin and streptomycin), an antifungal agent (for example, amphotericin), and a buffer (for example, a Good's buffer such as HEPES).

As the medium used in the pre-culturing (first culturing) and additional culturing (second culturing), any one of a serum-containing medium or a serum-free medium may be used.

When using a serum-containing medium, for example, fetal bovine serum (FBS) can be used as the serum. In addition, for easy separation of exosomes, FBS from which exosomes are removed can also be used. Examples of a commercially available exosome-removed medium can include FBS exosome-depleted and OneShot format (Gibco (registered trademark), Thermo Fisher Scientific Inc.). The concentration of the serum in the medium is, for example, 0.5 to 25% (v/v), 1 to 25% (v/v), 1 to 20% (v/v), 1 to 150 (v/v), 2 to 150 (v/v), 2 to 100 (v/v), 3 to 100 (v/v), 3 to 8% (v/v), or 3 to 5% (v/v). In one specific aspect, the concentration of the serum in the medium is 3% (v/v).

When using a serum-free medium, a serum replacement may be added. Examples of the serum replacement include bovine serum albumin (BSA), KnockOut Serum Replacement (KSR), and human serum albumin (HSA or human albumin, serum; HAS). As the human serum albumin, human serum albumin separated from human plasma or human serum albumin purified from rice expressing the human serum albumin gene (FUJIFILM Wako Pure Chemical Corporation) can be used. In general, factors such as a growth factor (hEGH or the like), a cytokine, and a hormone are further added to the medium. Examples of these added factors include epidermal growth factor (EGF), insulin, transferrin, hydrocortisone 21-hemisuccinate or a salt thereof, and dexamethasone (Dex), N2 supplement (FUJIFILM Wako Pure Chemical Corporation), NS supplement (FUJIFILM Wako Pure Chemical Corporation), and B-27 Serum Free Supplement (Thermo Fisher Scientific Inc., but the factors are not limited thereto.

A culture vessel used for the culturing is not particularly limited as long as it is suitable for adhesion culture, and examples thereof include a dish, a petri dish, a dish for tissue culture, a multidish, a microplate, a microwell plate, a multiplate, a multiwell plate, a chamber slide, a petri dish, a tube, a tray, and a culture bag. For performing suspension culture of the cells, it is possible to use a culture vessel with the surface processed so that the cells do not adhere thereto. Alternatively, in the case of adhesion culture, it is possible to use a culture vessel having the inner surface coated with a substrate for supporting cells for the purpose of improving adhesion of the cells. Examples of the substrate for supporting cells include collagen, gelatin, Matrigel, poly-L-lysine, laminin, and fibronectin. A preferred substrate for supporting cells is collagen or Matrigel.

The ectodermal cells which serve as the raw material can be seeded in the culture vessel at a cell density of, for example, 1 × 10² to 1 × 10⁶ cells/cm², 1 × 10³ to 1 × 10⁵ cells/cm², or 1 × 10³ to 1 × 10⁴ cells/cm².

During the culture, conditions that are generally applied when culturing ectodermal cells can be applied as they are. For the culture, a CO₂ incubator can be used, and in this case, any culture temperature and CO₂ concentration may be used as long as they are generally used, and the culture temperature and the CO₂ concentration can be, for example, 37°C and 5% (v/v).

The culturing period is a period during which the cells are cultured together with YA, and the period may be a continuous period or a discontinuous period, that is, a period that is the sum of a plurality of discontinuous periods. The culturing period should be longer than 28 days, and can be, for example, 5 weeks or longer, 6 weeks or longer, or 7 weeks or longer. The upper limit of the culturing period can be, for example, about 3 months or 100 days. Passaging can be suitably performed in accordance with the cell density in the culture vessel, the condition of the medium, or the condition of the cells, and the passage interval can be, for example, about 2 to 20 days.

The highly proliferating cells obtained by the present production method (in the present specification, may also be referred to as "HP cells") are defined as cells with increased proliferative ability, and are cells having higher cell proliferative activity and having the property to proliferate within a shorter amount of time, that is, the property of having shorter cell doubling time, the property to proliferate over a longer period of time, or the property that satisfy both of these requirements, compared to control ectodermal cells which are cultured under the same culturing conditions as those for the highly proliferating cells, except for using a medium not containing the small-molecule signaling pathway inhibitor. The cell populations contained in the culture obtained by the present production method can include, in addition to the HP cells, cells of which the proliferative ability has not been increased, thus having the proliferative ability that has not changed from that of the original cells, in other words, non-highly proliferating cells (in the present specification, may also be referred to as "non-HP cells"). The proportion of the HP cells in the cell populations obtained by the present production method may be 50% or higher, 60% or higher, 70% or higher, 80% or higher, 90% or higher, or 95% or higher, in terms of the number of the cells.

The highly proliferating cells can have the proliferative ability that allows the number of the highly proliferating cells after more than 28 days of the culturing period during which the cells are brought into contact with the inhibitor (in the present specification, referred to as "period T") (in the present specification, referred to as "the number N of the highly proliferating cells") to be greater than 1.0 times the number of ectodermal cells which have been cultured under the same culturing conditions, except that the cells are not brought into contact with the inhibitor, for the same culturing period (in the present specification, referred to as "the number N' of ectodermal cells"). In this case, the culturing condition can be, for example, a seeding density of 4 × 10³ cells/cm², culturing temperature of 37°C, and a CO₂ concentration of 5%. As a medium used when confirming the number N of the highly proliferating cells, any of the media that can be used for the pre-culturing and the additional culturing described above can be used. As two types of media used when confirming each of the number N of the highly proliferating cells and the number N' of ectodermal cells, media having the same compositions except for the presence or absence of the inhibitor can be used.

In one aspect, the period T is, for example, 90 days, 80 days, 70 days, 60 days, 50 days, 45 days, 42 days, 40 days, 35 days, 32 days, 30 days, or 29 days.

In one aspect, the number N of the highly proliferating cells is not particularly limited as long as the number is greater than 1.0 times the number N' of ectodermal cells, and may be, for example, equal to or greater than 1.1 times the number N' of cells, equal to or greater than 1.2 times the number N' of cells, equal to or greater than 1.3 times the number N' of cells, equal to or greater than 1.4 times the number N' of cells, or equal to or greater than 1.5 times the number N' of cells. The number N of the highly proliferating cells is preferably equal to or greater than 1.5 times the number N' of ectodermal cells.

The highly proliferating cells may be identified by the expression of a gene specific to the cells, that is, a marker gene. The expression of the marker gene for the highly proliferating cells may be confirmed based on either the gene or protein, as described above in the present specification. The method for confirming the expression of the gene or protein can be performed by the presence or absence of the expression, the specific expression level based on a measurement method, or comparison of the expression level with that of the same gene or protein in specific cells.

In the present specification, expression of a gene or protein may be expressed as being "positive" or "negative". Regarding the expression of a gene or protein, "positive" means that the expression of the gene or protein of interest has been confirmed, whereas "negative" means that the expression of the gene or protein of interest not been confirmed. Regarding the expression of a gene or protein, when the expression of "expression is high" or "positive", or "expression is low" or "negative" is used, it is possible to use specific cells that are different from the highly proliferating cells, as the subjected to be compared. The specific cells to be compared and contrasted with, that is, "control cells", may be cells in which the expression of the gene or protein of interest is extremely low, may be the ectodermal cells which serve as the raw material, that is, ectodermal cells not brought into contact with the inhibitor, or may be mature cells or the progenitor cells thereof.

For example, when the highly proliferating cells are "positive" for the expression of a marker gene, it can indicate that the expression level of the marker gene after the contact with the small-molecule signaling pathway inhibitor is equal to or greater than 1.0 times the expression level of the marker gene in the ectodermal cells that have been cultured without contacting the inhibitor. On the other hand, when the highly proliferating cells are "negative" for the expression of the marker gene, it can indicate that the expression level of the marker gene after the contact with the inhibitor is lower than 1.0 times the expression level of the marker gene in the ectodermal cells that have been cultured without contacting the inhibitor.

The culture conditions for the highly proliferating cells to be subjected to the method for confirming the expression level can be the same as those of the ectodermal cells that are not brought into contact with the inhibitor except for whether or not the cells are brought into contact with the inhibitor. For example, based on the condition of the cells, proliferating properties, the number of cells, and the like, suitable adjustments of conditions can be performed such as the same culturing period, the same cell passage numbers, and similar cell densities, so that the cells of interest have the same or very similar conditions, and those skilled in the art can select the culturing conditions based on the condition of the cells.

Here, the expression level of the marker gene after a certain amount of culturing period or longer during which the cells are brought into contact with the small-molecule signaling pathway inhibitor is expressed as the expression level relative to that of a control gene. As the control gene, for example, beta-actin (ACTB) or glyceraldehyde 3-phosphate dehydrogenase (GAPDH) can be used.

In one aspect, the highly proliferating cells can express at least one gene specific to mature cells at the same level as or at a higher level than the ectodermal cells which serve as the raw material, and can express at least one gene specific to progenitor cells at the same level as or at a higher level than the ectodermal cells which serve as the raw material.

In the present specification, the "progenitor cells" refer to cells at a differentiation stage which leads to mature cells, and are cells that develop from stem cells and differentiate into terminally differentiated cells that constitute the body.

Examples of the gene specific to mature cells, such as a gene expressed at a relatively high level in astrocytes or the like, can include GFAP, S100B, and SLC1A2. Examples of the gene specific to progenitor cells, such as genes expressed at a relatively high level in oligodendrocyte progenitor cells, neuroepithelial cells, and radial glial cells, can include NG2, Notch1, Nestin, and SOX2.

As long as the cells have high proliferative ability as described above, the highly proliferating cells may be cells characterized by any marker gene expression patterns; in other words, a cell population including cells having any characteristics. The highly proliferating cells may exhibit the expression patterns of the marker genes described later. With these marker gene expression patterns, the highly proliferating cells according to the present disclosure can be rapidly identified and extracted.

The highly proliferating cells can include cells negative for at least one selected from the group consisting of Musashi1 (MSI1), Notch1, Nestin, and SOX2.

For example, the highly proliferating cells can include GFAP-positive cells. In another aspect, the highly proliferating cells can include cells positive for GFAP and positive for NG2, or can express GFAP at the same level as or at a higher level than the ectodermal cells which serve as the raw material and express NG2 at the same level as or at a higher level than the ectodermal cells which serve as the raw material. In still another aspect, the highly proliferating cells can include cells positive for GFAP and S100B and positive for NG2, or can express GFAP and S100B at the same level as or at a higher level than the ectodermal cells which serve as the raw material and express NG2 at the same level as or at a higher level than the ectodermal cells which serve as the raw material.

In still another aspect, the highly proliferating cells can include cells expressing at least one gene specific to mature cells at the same level as or at a higher level than the ectodermal cells which serve as the raw material, and expressing at least one gene specific to progenitor cells at the same level as or at a higher level than the ectodermal cells which serve as the raw material.

In one aspect, the highly proliferating cells can include cells positive for NG2, or can include cells expressing NG2 at higher expression level than the ectodermal cells that have not been brought into contact with small-molecule signaling pathway inhibitor. That is, in this aspect, the expression level of NG2 in the highly proliferating cells may be higher than the expression level of NG2 in the ectodermal cells that have not been brought into contact with the small-molecule signaling pathway inhibitor.

For example, the highly proliferating cells can include cells positive for S100B and positive for NG2, or can express S100B at the same level as or at a higher level than the ectodermal cells which serve as the raw material and express NG2 at the same level as or at a higher level than the ectodermal cells which serve as the raw material.

In one aspect, the above-described cells included in the highly proliferating cells are negative for at least one selected from the group consisting of Musashi1 (MSI1), Notch1, Nestin, and SOX2. That is, in the cells included in the highly proliferating cells, the expression level of at least one marker gene selected from the group consisting of Musashi1, Notch1, Nestin, and SOX2 after the contact with the inhibitor can be lower than 1.0 times the expression level of the marker gene in the ectodermal cells that have been cultured without being brought into contact with the small-molecule signaling pathway inhibitor. In a preferred aspect, the above-described cells included in the highly proliferating cells are negative for two or more, three or more, or all selected from the group consisting of Musashi1, Notch1, Nestin, and SOX2.

In one aspect, the expression level in the cells negative for at least one selected from the group consisting of Musashi1, Notch1, Nestin, and SOX2 can be expressed as an expression level relative to a control gene. In this case, being "negative" means that, when, for example, beta-actin (ACTB) or glyceraldehyde 3-phosphate dehydrogenase (GAPDH) is used as the control gene, the expression level is lower than 1.0 times the expression level of the control gene.

As for the cell population of the highly proliferating cells according to the present disclosure, when the cells are highly proliferating cells having high proliferative ability, especially highly proliferating ectodermal cells, the cell population may be a heterogeneous cell population including both mature cells and progenitor cells regardless of the differentiation stage, or may be a cell population in which a plurality of types of ectodermal cells at relatively close differentiation stages occupy a very large majority.

In one aspect, the cell population of the highly proliferating cells obtained by the present production method may be a population including highly proliferating ectodermal progenitor cells as the main constituent or a population including both highly proliferating ectodermal progenitor cells and highly proliferating ectodermal mature cells. Here, the "main constituent" in the cell population refers to cells occupying at least 50% of the cell population in terms of the number of cells. In the present specification, "a very large majority" means that the number of the cells of interest in the cell population is 95% or more, 96% or more, 97% or more, 98% or more, 99% or more, or 99.5% or more, in terms of the number of cells.

The proportion of the ectodermal mature cells in the cell population of the obtained highly proliferating cells may be less than 50%, less than 40%, less than 30%, less than 20%, less than 10%, or less than 5%, in terms of the number of cells. Therefore, the present disclosure includes a method for enriching ectodermal cells with increased cell proliferative ability, the method including brining the ectodermal cells which serve as the raw material into contact with the small-molecule signaling pathway inhibitor. The contact between the ectodermal cells which serve as the raw material and the small-molecule signaling pathway inhibitor can be performed in vitro.

In the present production method, in a case where the ectodermal cells which serve as the raw material are astrocytes, the cell population of the highly proliferating cells may include an ectodermal cell lineage related to astrocytes. Examples of the cells of the ectodermal cell lineage related to astrocytes include astrocytes, radial glial cells, oligodendrocyte progenitor cells (also referred to as polydendrocyte), oligodendrocytes, and neuroepithelial cells. In addition, the cell population of the ectodermal cell lineage related to astrocytes may also include cells that have some of the characteristics exhibited by ectodermal cells and cannot be classified as any of the above-described cells, as long as the cell population exhibits increased cell proliferative ability.

Another aspect of the present disclosure relates to highly proliferating cells. The present highly proliferating cells may be the highly proliferating cells obtained by the production method described above or may be highly proliferating cells obtained by other production methods.

The highly proliferating cells according to one aspect of the present disclosure are highly proliferating cells having characteristics of ectodermal cells and proliferative ability that allows the number of the highly proliferating cells after more than 28 days of a culturing period during which the cells are brought into contact with a small-molecule signaling pathway inhibitor to be greater than 1.0 times the number of ectodermal cells which are cultured under the same culturing conditions, except that the cells are not brought into contact with the small-molecule signaling pathway inhibitor, for the same culturing period. As described above, by bringing the small-molecule signaling pathway inhibitor intro contact with the ectodermal cells, highly proliferating cells with increased cell proliferative ability can be obtained without gene transfer or genetic modification in the ectodermal cells. Accordingly, compared to the inhibitor non-contact group, the cells can be cultured for a longer period in the inhibitor contact group. As for the number of the obtained cells, the number of the cells is greater in the inhibitor contact group than in the inhibitor non-contact group.

In one aspect, when a cell group obtained by culturing human primary mature astrocytes by bringing the astrocytes into contact with a combination of a TGFβ receptor inhibitor and a ROCK inhibitor (inhibitor contact group) is compared with a cell group obtained by culturing the human primary mature astrocytes without bringing the astrocytes into contact with the combination of the inhibitors (inhibitor non-contact group), culturing can be performed for a longer period in the inhibitor contact group. In other words, compared to the inhibitor non-contact group, the cell proliferation continues for a longer period in the inhibitor contact group. Furthermore, regarding the final number of cells obtained by the proliferation, the number of cells in the inhibitor contact group is, for example, equal to or greater than 2 times, equal to or greater than 5 times, equal to or greater than 10 times, equal to or greater than 50 times, equal to or greater than 100 times, equal to or greater than 200 times, or 400 times the number of cells in the inhibitor non-contact group.

As described above, since the obtained highly proliferating cells have increased cell proliferative ability, a large number of cells can be obtained because of long-term culturing. Since the highly proliferating cells can produce a cell-secreted product as described below, the amount of the cell-secreted product that can be collected can also be increased in a case where a large number of cells can be obtained from the long-term culturing.

Regarding the highly proliferating cells according to the present aspect, the features described for the resultant products of the present production method apply as they are. However, as long as the cells have the characteristics of ectodermal cells and the proliferative ability that allows the number of the cells after more than 28 days of a culturing period during which the cells are brought into contact with the inhibitor to be greater than 1.0 times the number of ectodermal cells which are cultured under the same culturing conditions, except that the cells are not brought into contact with the inhibitor, for the same culturing period, the cells are not limited to the resultant products of the present production method.

As described above, the highly proliferating cells of the present disclosure can include cells positive for at least one selected from the group consisting of GFAP, NG2, and S100B. The highly proliferating cells can include, for example, at least one selected from the group consisting of the following cells (I) to (VII):
(I) GFAP-positive cells, for example, cells that express GFAP at the same level as or at a higher level than the ectodermal cells which serve as the raw material;
(II) NG2-positive cells, for example, cells that express NG2 at the same level as or at a higher level than the ectodermal cells which serve as the raw material;
(III) S100B-positive cells, for example, cells that express S100B at the same level as or at a higher level than the ectodermal cells which serve as the raw material;
(IV) cells that are positive for GFAP and positive for NG2, for example, cells that express GFAP at the same level as or at a higher level than the ectodermal cells which serve as the raw material and express NG2 at the same level as or at a higher level than the ectodermal cells which serve as the raw material;
(V) cells that are positive for GFAP and positive for S100B, for example, cells that express GFAP at the same level as or at a higher level than the ectodermal cells which serve as the raw material and express S100B at the same level as or at a higher level than the ectodermal cells which serve as the raw material;
(VI) cells that are positive for S100B and positive for NG2, for example, cells that express S100B at the same level as or at a higher level than the ectodermal cells which serve as the raw material and express NG2 at the same level as or at a higher level than the ectodermal cells which serve as the raw material; and
(VII) cells that are positive for GFAP, positive for S100B, and positive for NG2, for example, cells that express GFAP at the same level as or at a higher level than the ectodermal cells which serve as the raw material, express S100B at the same level as or at a higher level than the ectodermal cells which serve as the raw material, and express NG2 at the same level as or at a higher level than the ectodermal cells which serve as the raw material.

The highly proliferating cells of the present disclosure can include cells positive for SLC1A2. Specifically, the highly proliferating cells can include cells positive for at least one of the above genes and positive for SLC1A2.

In a case where the highly proliferating cells of the present disclosure include cells positive for at least GFAP, it is preferable that the expression level of the GFAP gene after being brought into contact with the small-molecule signaling pathway inhibitor is equal to greater than 1.0 times and less than 4.0 times the expression level of the GFAP gene in the ectodermal cells cultured without being brought into contact with the inhibitor.

The highly proliferating cells of the present disclosure can include cells positive for at least one selected from the group consisting of GFAP, NG2, and S100B and negative for at least one selected from Musashi1, Notch1, Nestin, and SOX2. That is, in this aspect, the cells of (I) to (VII) above are negative for at least one selected from Musashi1, Notch1, Nestin, and SOX2.

For the isolation of the highly proliferating cells, a known method for isolating specific cells can be adopted. For example, a fluorescence-activated cell sorting (FACS) method based on the expression of the proteins derived from the above-described marker genes and a magnetic cell separation method using dynabeads or the like can be used. Since the proliferative ability of the obtained highly proliferating cells is high, the highly proliferating cells may be practically isolated by performing long-term culturing which results in a cell population in which the proportion of the highly proliferating cells is very high.

### <2> Ectodermal Progenitor Cells and Production Method Thereof

The highly proliferating cells obtained by the production method according to the present disclosure may include cells exhibiting a characteristic of a more immature differentiation stage than the starting material ectodermal cells which serve as the raw material. Such ectodermal cells at an immature stage, in other words, ectodermal progenitor cells have higher cell proliferative activity, and have the property to proliferate within a shorter amount of time, the property to proliferate over a longer period of time, or the property that satisfy both of these requirements, compared to control ectodermal cells which are cultured under the same culturing condition as those for the ectodermal progenitor cells, except for using a medium not containing the inhibitor. In the present specification, these ectodermal progenitor cells exhibiting a highly proliferating property may be referred to as highly proliferating progenitor cells. The "highly proliferating cells" in the present specification may include the highly proliferating progenitor cells. Accordingly, there is an advantage in that the ectodermal cells at an immature stage can be grown over a longer period of time, whereby, for example, a cell-secreted product produced by the ectodermal progenitor cells can be obtained at a large amount within a shorter period of time compared to the control ectodermal cells.

Another aspect of the present disclosure relates to a production method for ectodermal progenitor cells, the production method including:
(i) preparing ectodermal cells which serve as a raw material,
(ii) culturing, for a period of more than 28 days, the ectodermal cells which serve as the raw material in a medium containing a small-molecule signaling pathway inhibitor by bringing the inhibitor into contact with the ectodermal cells, and
(iii) after the contact, performing additional culturing in a medium containing the inhibitor to obtain a culture containing highly proliferating cells that have higher cell proliferative ability than the ectodermal cells which serve as the raw material, in which the ectodermal progenitor cells are the highly proliferating cells. The production method for the ectodermal progenitor cells of the present disclosure may further include isolating the highly proliferating cells from the culture.

As the ectodermal cells which serve as the raw material and the small-molecule signaling pathway inhibitor in the production method for ectodermal progenitor cells of the present disclosure, for example, each of the ectodermal cells which serve as the raw material and the small-molecule signaling pathway inhibitor described in relation to the production method for highly proliferating cells of the present disclosure can be used.

For the isolation of the highly proliferating progenitor cells, a known method for isolating specific cells can be adopted. For example, a fluorescence-activated cell sorting (FACS) method based on the expression of the proteins derived from the above-described marker genes specific to progenitor cells and a magnetic cell separation method using dynabeads or the like can be used. Since the proliferative ability of the obtained highly proliferating progenitor cells is high, the highly proliferating progenitor cells may be practically isolated by performing long-term culturing which results in a cell population in which the proportion of the highly proliferating progenitor cells is very high.

In a case where the starting material ectodermal cells which serve as the raw material include astrocytes that have reached the terminal differentiation stage, the highly proliferating cells obtained by the production method according to the present disclosure may include cells exhibiting a characteristic of a more immature differentiation stage than the astrocytes. In addition to having increased proliferative ability, such ectodermal cells at an immature stage can also have the following characteristic of (a) or (b):
(a) compared to astrocytes, the expression level of the combination of GFAP and S100B; the combination of GFAP and SLC1A2; the combination of S100B and SLC1A2; the combination of GFAP and NG2; the combination of NG2 and SLC1A2; or the combination of NG2 and S100B is increased.
(b) compared to astrocytes, the expression level of the combination of GFAP, S100B, and NG2; the combination of GFAP, SLC1A2, and NG2; the combination of S100B, SLC1A2, and NG2; or the combination of GFAP, S100B, SLC1A2, and NG2 is increased.

Still another aspect of the present disclosure relates to the ectodermal progenitor cells according to the present disclosure and use thereof.

The ectodermal progenitor cells obtained by the above production method can be used in, for example, a method for evaluating a neuropathy therapeutic agent. Specifically, a candidate agent for the neuropathy therapeutic agent can be brought into contact with the ectodermal progenitor cells, whereby the usefulness of the candidate agent for the neuropathy therapeutic agent can be evaluated. Therefore, the present disclosure also relates to a method for evaluating a neuropathy therapeutic agent, the method including bringing a candidate agent for a neuropathy therapeutic agent into contact with the ectodermal progenitor cells. Furthermore, the present disclosure also relates to a method for evaluating a neurological disease model, the method including using the ectodermal progenitor cells described above.

The present disclosure further relates to a production method for ectodermal mature cells, the production method including inducing differentiation of the ectodermal progenitor cells obtained by the production method according to the present disclosure under a maturing condition, so as to obtain ectodermal mature cells.

Here, "under a maturing condition" refers to a culturing condition in which a known differentiation-inducing factor is used. Examples of the differentiation-inducing factor include fibroblast growth factor-3 (FGF-2); IL-6 family cytokines such as leukemia inhibitory factor (LIF) and ciliary neurotrophic factor (CNTF); bone morphogenetic protein (BMP) family cytokines such as BMP2 and BMP4, Notch family proteins; Wnt gene family proteins; sonic hedgehog protein; and retinoic acid. In addition, examples of a factor that is required for proliferation and maintenance of mature cells can include nerve growth factor (NGF), brain-derived neurotrophic factor (BDNF), neurotrophin-3 (NT-3), neurotrophin-4/5 (NT-4/5), and platelet-derived growth factor (PDGF).

As the culturing condition in which a differentiation-inducing factor is used, a known differentiation-inducing condition adopted in a method for inducing differentiation by a differentiation-inducing factor can be adopted, and the differentiation-inducing condition can be suitably selected in accordance with the type of the differentiation-inducing factor to be used.

### <3> Use of Inhibitor

The inhibitor used in the production method described above can be used for maintaining or promoting proliferative ability of ectodermal cells. Therefore, still another aspect of the present disclosure relates to a proliferating property regulating agent for ectodermal cells, the regulating agent containing a small-molecule signaling pathway inhibitor, for example, at least one selected from the group consisting of a TGFβ receptor inhibitor and a ROCK inhibitor. Specifically, the present disclosure relates to a proliferating property regulating agent which is used for the maintenance or promotion of the proliferative ability of the highly proliferating cells obtained by the above-described production method or ectodermal cells. Here, the term "proliferating property regulation" in the present specification can refer to achieving at least one of maintenance of cell proliferation or promotion of proliferation. The terms "maintenance of proliferation" and "promotion of proliferation" are not particularly distinguished, as long as the number of cells is maintained or increased over the cell culturing period. As the concentration of the proliferating property regulating agent to be used for regulating the proliferating property of cells, the concentrations described above regarding the concentration used in the above-described production method can be adopted as they are.

The present disclosure also relates to a culturing method for the highly proliferating cells obtained by the above-described production method or ectodermal cells, the culturing method including passaging the cell population or the cells in the presence of a small-molecule signaling pathway inhibitor, for example, at least one inhibitor selected from the group consisting of a TGFβ receptor inhibitor and a ROCK inhibitor. Here, the "culturing method" includes a method of regulating the proliferating property of the highly proliferating cells or ectodermal cells. As the concentration of the inhibitor to be used in the case of using the present culturing method, the concentrations described above regarding the concentration used in the above-described production method can be adopted as they are.

### <4> Cell-Secreted Product and Production Method Thereof

Still another aspect of the present disclosure is a production method for a cell-secreted product, the production method including obtaining a culture containing a cell-secreted product secreted from the highly proliferating cells according to the present disclosure and separating the cell-secreted product from the culture. The present production method may further include any steps as necessary. Since the highly proliferating cells according to the present disclosure have increased cell proliferative ability, the highly proliferating cells proliferate within a shorter amount of time, proliferate over a longer period of time, or proliferate within a shorter amount of time and proliferate over a longer period of time, compared to the control cells which are cultured under the same culturing condition as those for the highly proliferating cells, except for using a medium not containing the inhibitor. Therefore, in the present production method for a cell-secreted product, the cell-secreted product secreted by the highly proliferating cells can be obtained in a large amount within a shorter period of time, compared to the case of using the control cells.

The cell-secreted product is also referred to as a secretome, and is not particularly limited as long as it is a substance secreted from cells. Examples of the cell-secreted product include extracellular vesicles such as exosomes, microvesicles, and apoptotic vesicles; and functional proteins such as cytokines, hormones, and antibodies. In one embodiment, the cell-secreted product may be extracellular vesicles, particularly, exosomes.

The culture prepared in the production method for a cell-secreted product according to the present disclosure contains the cell-secreted product secreted from the highly proliferating cells obtained from any of the aspects described above. The culture containing the cell-secreted product may be produced by performing the production method for highly proliferating cells according to the present disclosure, or may be obtained by acquiring and culturing the highly proliferating cells according to the present disclosure. The culture may also be separately acquired by performing the production method according to the present disclosure, or may be the culture of the highly proliferating cells according to the present disclosure being separately acquired. In the present specification, the "culture" refers to the combination of the cultured cells and culture supernatant (conditioned medium) obtained by culturing the cells.

The production method for a cell-secreted product according to the present disclosure includes separating the cell-secreted product from the culture. As the method for separating the cell-secreted product from the culture, for example, a known separation method such as removal of the supernatant and centrifugation can be used. The cell-secreted product separated from the culture may also be obtained in a form of a composition containing the cell-secreted product. In a case where the cell-secreted product is a composition containing the cell-secreted product, that is, a cell-secreted product-containing composition, the cell-secreted product-containing composition can contain the cell-secreted product and an aqueous vehicle. The aqueous vehicle is not particularly limited as long as it is a known aqueous vehicle selected in accordance with the type of the cell-secreted product or the like, and examples thereof can include a culture supernatant, water, a buffer (for example, a phosphate buffer and a Good's buffer), saline, and a medium.

The production method for a cell-secreted product can further include purifying or isolating the separated cell-secreted product. As a purification method or isolation method for the cell-secreted product, a known method can be adopted in accordance with the type of the cell-secreted product, without any particular limitation.

Examples of the isolation or purification method for the cell-secreted product can include filtration and concentration. For example, the cell-secreted product can be isolated or purified by performing filtration using a membrane having a size or molecular weight cutoff value. As another method, for example, the cell-secreted product may be filtered or concentrated using tangential flow filtration or ultrafiltration. The cell-secreted product can be used in the form of the composition containing the cell-secreted product or in the form of the isolated cell-secreted product obtained after the isolation or purification. The cell-secreted product can be stored, refrigerated, or cryopreserved in the form of the composition or isolated cell-secreted product as it is, or by subjecting the composition or isolated cell-secreted product to a known treatment such as spray drying or freeze drying treatment.

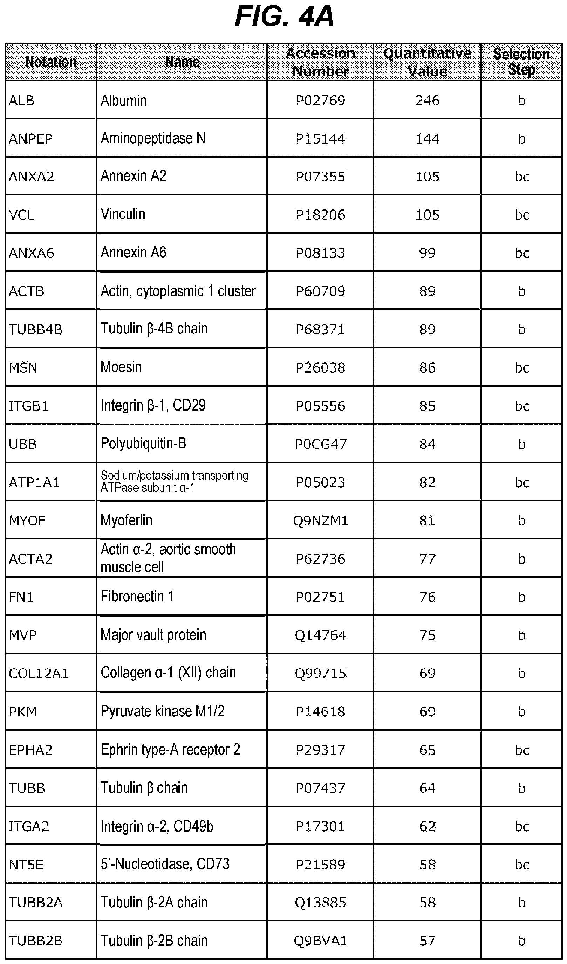

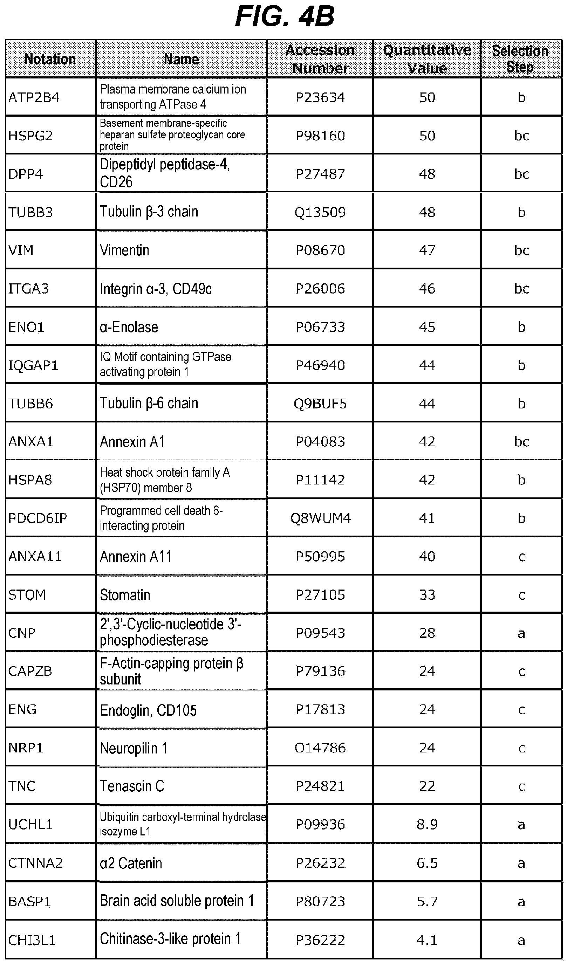

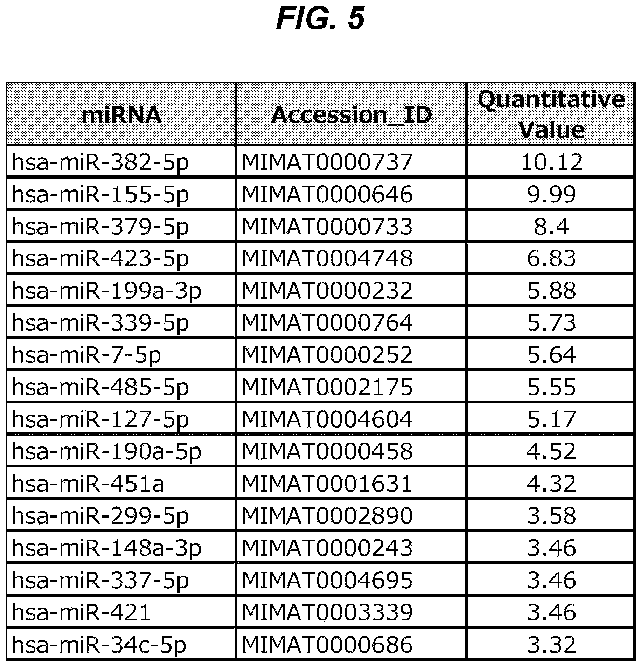

Specifically, the cell-secreted product according to the present disclosure contains a protein and/or miRNA. The protein is selected from those shown in Figs. 4A and 4B, and the miRNA is selected from those shown in Figs. 5 to 10. In Figs. 4A and 4B, the description in the parentheses following each protein name refers to the accession number in UniProtKB/Swiss-Prot (https://www.uniprot.org/). In Figs. 5 to 10, the description in the parentheses following each miRNA name refers to the accession number in miRBase (Release 21) (https://www.mirbase.org/). The cell-secreted product may be any cell-secreted product as long as it contains at least one selected from these proteins or at least one selected from these miRNAs, and the cell-secreted product may contain at least one selected from these proteins and at least one selected from these miRNAs.

As the protein, in one embodiment, the cell-secreted product,
·PA group: may at least contain the combination of vinculin (P18206), integrin β-1, CD29 (P05556), pyruvate kinase M1/2 (P14618), and ephrin type-A receptor 2 (P29317),
·PB group: may at least contain the combination of vinculin (P18206), integrin β-1, CD29 (P05556), pyruvate kinase M1/2 (P14618), and ephrin type-A receptor 2 (P29317); and at least one selected from the group consisting of aminopeptidase N (P15144), annexin A2 (P07355), annexin A6 (P08133), myoferlin (Q9NZM1), and 5'-nucleotidase, CD73 (P21589)
·PC group: may contain the combination of vinculin (P18206), integrin β-1, CD29 (P05556), pyruvate kinase M1/2 (P14618), and ephrin type-A receptor 2 (P29317); the combination of aminopeptidase N (P15144), annexin A2 (P07355), annexin A6 (P08133), myoferlin (Q9NZM1), and 5'-nucleotidase, CD73 (P21589); and at least one selected from the group consisting of moesin (P26038), integrin α-2, CD49b (P17301), integrin α-3, CD49c (P26006), 2',3'-cyclic-nucleotide 3'-phosphodiesterase (P09543), F-actin-capping protein β subunit (P79136), neuropilin 1 (014786), and tenascin C (P24821), or
·PD group: may contain all of the proteins indicated in Figs. 4A and 4B.

As miRNA, in one embodiment, the cell-secreted product,
·RA group: may at least contain the combination of hsa-miR-382-5p (MIMAT0000737), hsa-miR-155-5p (MIMAT0000646), hsa-miR-379-5p (MIMAT0000733), hsa-miR-16-5p (MIMAT0000069), hsa-miR-382-5p (MIMAT0000737), hsa-miR-16-5p (MIMAT0000069), hsa-miR-382-5p (MIMAT0000737), hsa-miR-21-5p (MIMAT0000076), hsa-let-7a-5p (MIMAT0000062), hsa-miR-16-5p (MIMAT0000069), hsa-miR-409-3p (MIMAT0001639), hsa-let-7a-5p (MIMAT0000062), and hsa-let-7f-5p (MIMAT0000067),
·RB group: may at least contain the combination of hsa-miR-382-5p (MIMAT0000737), hsa-miR-155-5p (MIMAT0000646), hsa-miR-379-5p (MIMAT0000733), hsa-miR-16-5p (MIMAT0000069), hsa-miR-382-5p (MIMAT0000737), hsa-miR-16-5p (MIMAT0000069), hsa-miR-382-5p (MIMAT0000737), hsa-miR-21-5p (MIMAT0000076), hsa-let-7a-5p (MIMAT0000062), hsa-miR-16-5p (MIMAT0000069), hsa-miR-409-3p (MIMAT0001639), hsa-let-7a-5p (MIMAT0000062), and hsa-let-7f-5p (MIMAT0000067); and at least one selected from the group consisting of hsa-miR-151a-3p (MIMAT0000757), hsa-miR-93-5p (MIMAT0000093), hsa-miR-29a-3p (MIMAT0000086), hsa-miR-9-5p (MIMAT0000441), hsa-miR-25-3p (MIMAT0000081), hsa-miR-92a-3p (MIMAT0000092), hsa-miR-34a-5p (MIMAT0000255), hsa-miR-26a-5p (MIMAT0000082), hsa-miR-26b-5p (MIMAT0000083), hsa-miR-29a-3p (MIMAT0000086), hsa-miR-9-5p (MIMAT0000441), hsa-miR-155-5p (MIMAT0000646), hsa-miR-92a-3p (MIMAT0000092), hsa-miR-103a-3p (MIMAT0000101), and hsa-miR-26a-5p (MIMAT0000082),
·RC group: may at least contain the combination of hsa-miR-382-5p (MIMAT0000737), hsa-miR-155-5p (MIMAT0000646), hsa-miR-379-5p (MIMAT0000733), hsa-miR-16-5p (MIMAT0000069), hsa-miR-382-5p (MIMAT0000737), hsa-miR-16-5p (MIMAT0000069), hsa-miR-382-5p (MIMAT0000737), hsa-miR-21-5p (MIMAT0000076), hsa-let-7a-5p (MIMAT0000062), hsa-miR-16-5p (MIMAT0000069), hsa-miR-409-3p (MIMAT0001639), hsa-let-7a-5p (MIMAT0000062), and hsa-let-7f-5p (MIMAT0000067); the combination of hsa-miR-151a-3p (MIMAT0000757), hsa-miR-93-5p (MIMAT0000093), hsa-miR-29a-3p (MIMAT0000086), hsa-miR-9-5p (MIMAT0000441), hsa-miR-25-3p (MIMAT0000081), hsa-miR-92a-3p (MIMAT0000092), hsa-miR-34a-5p (MIMAT0000255), hsa-miR-26a-5p (MIMAT0000082), hsa-miR-26b-5p (MIMAT0000083), hsa-miR-29a-3p (MIMAT0000086), hsa-miR-9-5p (MIMAT0000441), hsa-miR-155-5p (MIMAT0000646), hsa-miR-92a-3p (MIMAT0000092), hsa-miR-103a-3p (MIMAT0000101), and hsa-miR-26a-5p (MIMAT0000082); and at least one selected from the group consisting of hsa-miR-128-3p (MIMAT0000424), hsa-miR-222-3p (MIMAT0000279), hsa-miR-31-5p (MIMAT0000089), hsa-miR-381-3p (MIMAT0000736), hsa-miR-128-3p (MIMAT0000424), hsa-miR-24-3p (MIMAT0000080), hsa-miR-26b-5p (MIMAT0000083), hsa-miR-24-3p (MIMAT0000080), hsa-miR-30a-3p (MIMAT0000088), hsa-miR-31-5p (MIMAT0000089), hsa-miR-128-3p (MIMAT0000424), hsa-miR-23a-3p (MIMAT0000078), hsa-miR-24-3p (MIMAT0000080), hsa-let-7d-5p (MIMAT0000065), and hsa-miR-654-3p (MIMAT0004814), or
·RD group: may contain all of the miRNAs indicated in Figs. 5 to 10.

In another embodiment, the cell-secreted product may contain, as the proteins, the protein group PA group, PB group, PC group, or PD group described above, and, as the miRNAs, the miRNA group RA group, RB group, RC group, or RD group described above. Therefore, the cell-secreted product according to one embodiment can be used as a pharmaceutical composition having a known action attributable to these proteins and miRNAs.

In one aspect, extracellular vesicles are vesicles having a lipid bilayer. The diameter of the extracellular vesicle is, for example, 50 nm to 5 µM, or 50 nm to 1,000 nm. The diameter of an exosome, which is a type of the extracellular vesicles, is, for example, 50 nm to 200 nm. Since exosomes contain various biologically active substances such as proteins, nucleic acids, sugars, and lipids, the exosomes are expected to be used in the treatment and diagnosis of diseases, pharmaceutical products, cosmetics, and the like.

Exosomes are secreted from adherent cells into the culture, in particular, into the culture supernatant. In addition, when non-adherent cells are present in a cell suspension, exosomes are secreted from the non-adherent cells into the culture supernatant or the cell suspension. The exosomes derived from the adherent cells or non-adherent cells are not particularly limited, as long as the exosomes are obtained from adherent ectodermal cells or non-adherent ectodermal cells. Examples of the exosomes include the exosomes described in Journal of Controlled Release vol. 323, pp. 225 to 239 (2020).

The exosomes can be isolated based on the molecular weight, size, shape, composition, or biological activity. Specifically, the isolation can be performed by fractionating a precipitate obtained by ultracentrifugation, fractionating a fraction obtained by density gradient ultracentrifugation, fractionating using size exclusion chromatography, fractionating using ion exchange chromatography (for example, CIMmultus^{™} EV (manufactured by Sartorius BIA Separations d.o.o.), fractionating by capture using a protein (for example, MagCapture^{™} exosome isolation kit PS (FUJIFILM Wako Pure Chemical Corporation)), fractionating by capture using an antibody, fractionating a precipitate using a polymer such as polyethylene glycol, or the like. One of these methods or a combination of a plurality of these methods can be performed.

The properties of exosomes can be utilized to track the activity of exosomes in a method for producing exosomes as the cell-secreted product. For example, the activity of exosomes can be confirmed using static light scattering, dynamic light scattering, an ultraviolet-visible detector, a fluorescence detector, or a differential refractive index detector.

From the viewpoint of obtaining exosomes with high purity, when isolating or purifying exosomes, in general, the culture medium which contains the obtained highly proliferating cells can be replaced with a collection medium to perform additional culturing. The collection medium is, specifically, a medium for collecting a culture supernatant. The additional culturing refers to short-term culturing which is performed using the collection medium after the culturing using the culture medium. The duration of the additional culturing can be, for example, 6 hours or longer, 12 hours or longer, 18 hours or longer, 24 hours or longer, 36 hours or longer, 48 hours or longer, or 60 hours or longer, and can be, for example, 96 hours or shorter or 72 hours or shorter. By performing the additional culturing, the contamination rate of exosomes that are derived from a source different from the highly proliferating cells and can be included in the culture medium can be lowered, and the purity of exosomes derived from the target highly proliferating cells can be increased.

Examples of the collection medium can include a serum-free medium and a medium that has been subjected to an exosome-removal treatment. Examples of a commercially available medium that has been subjected to an exosome-removal treatment can include FBS exosome-depleted and OneShot format (Gibco (registered trademark), Thermo Fisher Scientific Inc.).

In the case of using the collection medium, exosomes can be obtained by performing centrifugation after the additional culturing, thus fractionating the culture supernatant as an exosome-containing composition, and subjecting the fractionated culture supernatant to the above-described isolation or purification method for exosomes as necessary.

### <5> Use of Highly Proliferating Cells and Cell-Secreted Product

The cell-secreted product separated from the highly proliferating cells according to one embodiment, for example, the exosomes separated or purified as described above, are expected to have various effects on various cell species, such as a neurite outgrowth suppressing effect, a neurite outgrowth effect, a neurite network-forming effect, a neuronal death prevention effect, and a neuron proliferation promoting effect. Based on these functions, usefulness of the cell-secreted product is expected as a pharmaceutical composition for preventing or treating a disorder associated with peripheral nerve cells or central nerve cells. For example, as for the differentiation of the peripheral nerve cells, the neurite outgrowth suppressing effect of the cell-secreted product separated from the highly proliferating cells can suppress the differentiation of a more undifferentiated cells into peripheral nerve cells, particularly, sympathetic nerve cells. Therefore, by using the cell-secreted product according to the present disclosure on peripheral nerve cells, suppression of the peripheral nervous system, in particular, the sympathetic nervous system can be expected. In addition, for example, by using the cell-secreted product separated from the highly proliferating cells on central nerve cells, activation of the central nervous system can be expected, based on the neurite outgrowth effect, the neurite network-forming effect, the neuronal death prevention effect, and the neuron proliferation promoting effect of the cell-secreted product.

Therefore, a pharmaceutical composition containing a therapeutically or prophylactically effective amount of the cell-secreted product separated from the highly proliferating cells and a pharmaceutically acceptable carrier, and a treatment or prevention method including administering the pharmaceutical composition to a subject are also included in the scope of the present disclosure. In the present specification, the term "treatment" includes not only radical cure of the disorder, but also improvement of the symptoms of the disorder, such as alleviation and remission.

The disorder to be treated or prevented may be any disorder as long as the disorder is associated with peripheral nerve cells or central nerve cells, and, for example, the disorder is selected from the group consisting of a cancer, pain, Alzheimer's disease, Parkinson's disease, depression, schizophrenia, and dementia. Examples of the disorder associated with peripheral nerve cells, in particular, sympathetic nerve cells, include a cancer and pain associated with the sympathetic nerve cells. The disorder associated with central nerve cells is selected from, for example, the group consisting of Alzheimer's disease, Parkinson's disease, depression, schizophrenia, and dementia.

The pharmaceutically acceptable carrier is not particularly limited, and a carrier known in the art is used. Examples of the carrier include saline.
Furthermore, in relation to the above effects, a sympathetic nervous system suppression method, a neurite outgrowth suppression method, and the like which include bringing the cell-secreted products secreted from the highly proliferating cells, specifically, exosomes, into contact with neurons such as sympathetic nerve cells are also included in the scope of the present disclosure.

In the present specification, a numerical range indicated using "to" indicates a range that includes the numerical values before and after "to" as the minimum value and maximum value, respectively. In the numerical ranges described in the present specification in stages, the upper limit or lower limit of the numerical range of one stage can be arbitrarily combined with the upper limit or lower limit of the numerical range of another stage.

The present disclosure is based on the following Japanese patent application and benefits from the priority right of the Japanese patent application. Furthermore, the entire contents of the following Japanese patent application are incorporated by reference into the present disclosure:
Japanese Patent Application No. 2021-124172 filed on July 29, 2021 entitled "METHOD FOR PRODUCING HIGHLY PROLIFERATIVE CELL, AND HIGHLY PROLIFERATIVE CELL AND USE THEREOF".

All publications, patent applications, and technical standards mentioned in this disclosure are incorporated by reference into the present disclosure in their entirety.

### Examples

### Example 1: Long-Term Culturing of Human Astrocytes Using Medium Containing Inhibitor

Long-term culturing of human astrocytes was performed using the following materials, reagents, and products for culturing.

### <Materials>

·Normal Human Astrocytes (NHA) (CC-2565, Lonza Group AG)

### <Reagents Used and Products for Culturing>

·Culture medium: AGM Astrocyte Growth Medium Bullet Kit (CC-3186, Lonza Group AG) (composition of AGM: basal medium, FBS (3% (v/v)), L-glutamine, ascorbic acid, hEGF, insulin, and antibiotic)
·Inhibitor Y: CultureSure (registered trademark) Y-27632 (034-24024, FUJIFILM Wako Pure Chemical Corporation), final concentration: 10 µM
·Inhibitor A: CultureSure (registered trademark) A-83-01(035-24113, FUJIFILM Wako Pure Chemical Corporation), final concentration: 0.5 µM

- Cell detachment agent: Accutase (AT104, Innovative Cell Technologies, Inc.)
- Cell buffer: phosphate buffered saline (Dulbecco's formula; without calcium and magnesium) (BNDSBN200, KAC Co., Ltd.)
- Medium for cell freezing: CELLBANKER 1 (CB011 TaKaRa Bio Inc. (NIPPON ZENYAKU KOGYO CO.,LTD.))
- Cell culturing dish 60 mm (150462, Thermo Fisher Scientific Inc.)
- Cell culturing dish 100mm (150466, Thermo Fisher Scientific Inc.)
- Cell culturing dish 150 mm (150468, Thermo Fisher Scientific Inc.)
- Stericup Quick Release-GP Sterile Vacuum Filtration System (S2GPU02RE, Merck Millipore)
- Inhibitor-free medium for culture supernatant collection: Dulbecco's Modied Eagle Medium (DMEM) (C11995500CP, Gibco (registered trademark), Thermo Fisher Scientific Inc.) and N-2 Supplement (x100) (17502-048, Gibco (registered trademark), Thermo Fisher Scientific Inc.)

### <Procedure for Long-Term Culturing>

The long-term culturing was performed in accordance with the following procedure. A frozen tube of the human astrocyte cells (NHA) was thawed in a water bath at 37°C. Next, the lysed cells were transferred to the culture medium so that the total volume was 10 mL and subjected to centrifugation at 180 × g for 3 minutes at room temperature, and then the medium was removed. The cells were divided into 4 groups; a group to which only the inhibitor Y was added (hereinafter, the NHA-Y group), a group to which only the inhibitor A was added (hereinafter, the NHA-A group), a group to which the inhibitor Y and the inhibitor A were added (hereinafter, the NHA-YA group), and a group to Ih no inhibitor was added (normal group). The cells were suspended in the culture medium again and seeded to a 6-well plate (multidish 6 well for cell culturing (140675, Thermo Fisher Scientific Inc.; hereinafter, omitted) at a seeding density of 4.2 × 10³ cells/cm². On the next day, the medium for each group was replaced with a culture medium containing each inhibitor (or not containing any inhibitor) (the concentration of each inhibitor was as described above) as indicated in Table 2, and the culturing was continued. During the culturing period, cells were observed using a microscope and photographs were taken.

The medium was replaced every other day or every two days with fresh culture medium containing each inhibitor (or not containing any inhibitor). When the confluency reached 80 to 90%, the cells were detached using the cell detachment agent, and the cells were suspended in the culture medium containing each inhibitor (or not containing any inhibitor) and passaged into a 6-well plate at a seeding density of 4.2 ×10³ cells/cm². While continuing the passaging, the culturing was performed for 41 days in the culture medium containing each inhibitor (or not containing any inhibitor).

The cell passage number and the number of days up to the day of final passage in each group are indicated in the following Table 1, and the correlations between the number of culturing days and the total number of cells are indicated in Table 2.

**[Table 1]**

| Inhibitor addition pattern | Passage number (times) | Number of days up to day of final passage (days) |
|---|---|---|
| Y | 5 | 29 |
| A | 4 | 30 |
| YA | 6 | 37 |
| Normal | 3 | 22 |

**[Table 2]**

| Number of culturing days (days) | Number of cells (× 10⁴ cells) | | | |
|---|---|---|---|---|
| | Y | A | YA | Normal |
| 0 | 8.00 | 8.00 | 8.00 | 8.00 |
| 5 | 87.25 | 55.75 | 28.00 | 104.50 |
| 8 | 245.39 | | | |
| 9 | | | 128.63 | |
| 13 | 391. 09 | | 751.65 | 182.88 |
| 15 | | 264.81 | | |
| 16 | | | | |
| 19 | 733.30 | | 5026.68 | |
| 20 | | | | |
| 22 | | | | 234.31 |
| 23 | | 595.83 | | |
| 26 | | | 22305.87 | |
| 27 | | | | |
| 29 | 756.21 | | | |
| 30 | | 1005.46 | | |
| 33 | | | | |
| 35 | | | | |
| 37 | | | 98285.24 | |

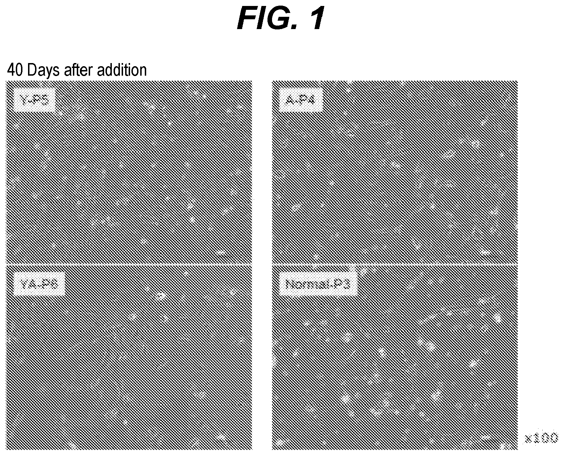

As shown in Table 1, it was found that, in the normal group, the proliferation stopped 22 days after the contact with the inhibitor, and, in the NHA-YA group, the cells continued to proliferate for 37 days after the contact when the inhibition was maximum. Furthermore, as for the number of cells obtained by the proliferation, the number of cells in the NHA-YA group was about 400 times the number of cells in the normal group, as can be seen from Table 2. In all experimental groups, the observation was continued after the day of the final passage up to Day 41 after the start of the culturing while performing the medium replacements. Fig. 1 shows the form of the cells on Day 41 after the start of the culturing (100x magnification). In Fig. 1, "P" indicates the passage number. For example, "Y-P5" refers to a cell group that has been passaged 5 times and has been cultured using the medium containing the inhibitor Y.

### Example 2: Collection and Identification of Exosomes in Culture Supernatant

### <Collection of Supernatant of Human Astrocyte Cell Culture>

In accordance with the following procedure, the culture supernatants obtained when the human astrocyte cells were cultured using a YA-containing culture medium or an inhibitor-free medium were collected.

### (1) Collection of Culture Supernatant in Culture of Human Astrocyte Cells in YA-Containing Culture Medium

The frozen human astrocyte cells were thawed as in Example 1 and washed with the medium once, and then the cells were suspended in the culture medium again and seeded in a 60-mm cell culturing dish at a seeding density of 3.5 × 10³ cells/cm². On the next day, the medium was replaced with the YA-containing culture medium, and the culturing was continued. When the confluency reached 80 to 90%, the cells were detached using the cell detachment agent, and the cells were suspended in the YA-containing culture medium and passaged into a 100-mm cell culturing dish at a seeding density of 3.5 ×10³ cells/cm². The group of cells cultured in the YA-containing culture medium is referred to as the NHA-YA group, as in Example 1.

After performing passaging 5 times and culturing the cells in the YA-containing culture medium for 60 days in total, a considerable number of cells were able to be secured in the NHA-YA group, and thus, seeding was performed on a 150-mm cell culturing dish at a seeding density of 8 × 10³ cells/cm². At this time, the number of seeded cells was 1.16 × 10⁶ cells per dish, and 6 dishes were used. Therefore, the total number of seeded cells was 6.96 × 10⁶ cells.

On the next day, the YA-containing culture medium was replaced with a collection medium at a volume of a 20 ml/150 mm dish, and additional culturing was started. Here, as the collection medium, a medium obtained by excluding only FBS from the YA-containing culture medium was used. Two days after the start of the additional culturing, the culture supernatant of the additional culture was collected from the dish, transferred to a tube, subjected to centrifugation at 2,000 × g for 10 minutes at 4°C, then filtered with a 0.22-µM filter system, and stored at 4°C. The cells remaining in the dish after collecting the culture supernatant were detached using the cell detachment agent, and the number of cells were counted. As a result, the total number of cells obtained as the NHA-YA group was 5.10 × 10⁶, and the residual ratio relative to the total number of seeded cells was 73.3%.

### (2) Collection of Culture Supernatant in Culture of Human Astrocyte Cells in Inhibitor-Free Culture Medium (Control)

The frozen human astrocyte cells were thawed as in Example 1 and washed with the medium once, and then the cells were suspended in the culture medium again and seeded in a 60-mm cell culturing dish at a seeding density of 3.5 × 10³ cells/cm². The group of cells cultured in a culture medium not containing an inhibitor is referred to as the normal group, as in Example 1.

In the normal group, after performing passaging twice and culturing the cells in the culture medium not containing an inhibitor for 26 days in total, seeding was performed on a 150-mm cell culturing dish at a seeding density of 8 × 10³ cells/cm². At this time, the number of seeded cells was 1.16 × 10⁶ cells per dish, and 2 dishes were used. Therefore, the total number of seeded cells was 2.32 × 10⁶ cells.

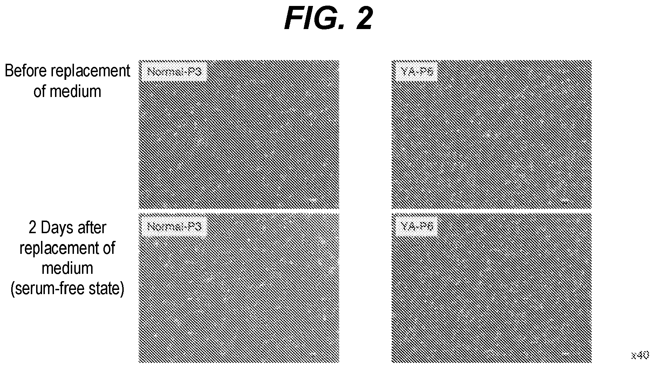

On the next day, the culture medium was replaced with a collection medium obtained by adding N-2 Supplement to DMEM at a volume of a 20 ml/150 mm dish, and additional culturing was started. Two days after the start of the additional culturing, the culture supernatant of the additional culture was collected from the dish, transferred to a tube, subjected to centrifugation at 2,000 × g for 10 minutes at 4°C, then filtered with a 0.22-µM filter system, and stored at 4°C. The cells remaining in the dish after collecting the culture supernatant were detached using the cell detachment agent, and the number of cells were counted. As a result, the total number of cells obtained as the normal group was 8.25 × 10⁵, and the residual ratio relative to the total number of seeded cells was 35.6%. The supernatant was centrifuged at 2,000 × g for 10 minutes at 4°C, and then filtered with a 0.22-µM filter system and stored at 4°C. The forms of the cells before and after replacing the medium to the collection medium are shown in Fig. 2.

### <Measurement of Number of Cells, Number of Particles, and Particle Size Distribution in Culture Supernatant>

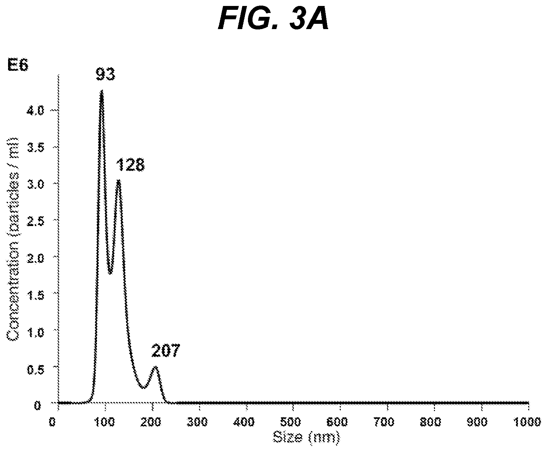

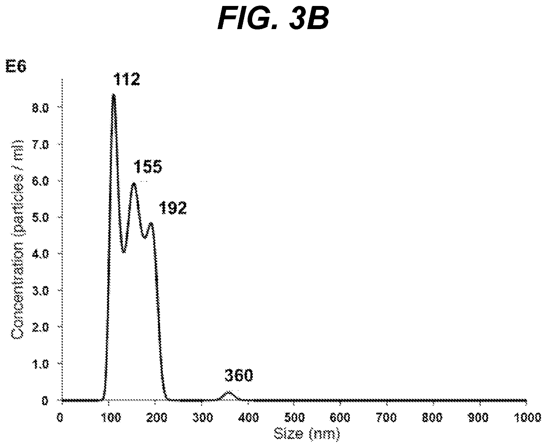

Each of the collected culture supernatants of the additional culture of the normal group and the NHA-YA group above was diluted 5-fold using the cell buffer (the buffer was filtered with a 0.22-µM filter before use), and then the number of particles and particle size distribution were confirmed with NanoSight LM10 (Malvern Panalytical Ltd). As the blank, the number of particles in the cell buffer was also measured, and the number of particles in the cell buffer was subtracted from the measurement values for each culture supernatant, so as to calculate the number of particles in the culture supernatant. The number of particles and particle size distribution were each confirmed twice. Examples of the number and sizes of particles in the collected culture supernatant of the additional culture are shown in Table 3, and the particle size distributions are shown in Figs. 3A and 3B, respectively. In Figs. 3A and 3B, the longitudinal axis represents the number of particles (10⁶ particles/mL), and the horizontal axis represents the particle size (nm).

**[Table 3]**

| Type of culture supernatant in additional culture | Number of particles (× 10⁸/mL) | Particle size (peak, nm) |
|---|---|---|
| Normal group | 7.54 | 93, 128, 207 |
| NHA-YA group | 27.10 | 112, 155, 192, 360 |

As is clear from Table 3 and Fig. 3B, multiple peaks can be observed in the particle size range of 100 nm to 200 nm in the culture supernatant of the NHA-YA group, indicating that exosomes were contained. In addition, it was confirmed that the amount of exosomes contained in the culture supernatant of the NHA-YA group was greater compared to the normal group. Then, by a purification method such as tangential flow filtration or ultrafiltration, particles with peaks in the particles size range of 100 nm to 200 nm were able to be isolated or purified, as described later.

### Example 3-1: Confirmation of Ectodermal Cell Markers by Quantitative PCR (1)

Expression levels of the following ectodermal cell marker genes were examined in the astrocyte cell group which was used as the starting material for the culturing in Example 1 and the obtained ectodermal cell population. Detection primers were prepared based on the sequences registered with the accession numbers in an NCBI database RefSeq, which are indicated below.
·Notch1 (including vX1) (NM_017617)
·Nestin (NM_006617)
·SOX2 (SRY-box transcription factor 2) (NM_003106)
·S100B (S100 calcium binding protein B) (NM_006272)
·NG2 (Chondroitin sulfate proteoglycan 4) (NM_001897)
·GFAP (Glial fibrillary acidic protein) (v1.v4, vX1, and vX3) (NM_002055)

### <Synthesis of cDNA>

RNA was extracted from the following samples in accordance with a conventional method, and cDNA was synthesized using the RNA. Preparation of the RNA samples and setting of the reaction conditions were performed in accordance with the protocol attached to the product.

### Samples:

·Normal human astrocytes (NHA) (purchased primary cultured cells were awakened, allowed to proliferate, and frozen, then awakened again and cultured for 4 days; passaging was not performed)
·NHA-YA (purchased primary cultured cells were awakened, allowed to proliferate, and frozen, then awakened again and cultured in a medium containing YA for 28 days, then frozen, and then awakened again and cultured in a medium containing YA for 14 days; during the culturing period of 14 days, passaging was performed once)
Reagent: High-Capacity cDNA Reverse Transcription Kit with RNase Inhibitor (Applied Biosystems, Inc.)
Device: SimpliAmp Thermal Cycler (Applied Biosystems, Inc.)

### <Quantitative PCR>

The detection primers prepared are shown in the following Table 4. Using these primers, the expression of the ectodermal cell marker genes was examined by quantitative PCR. Preparation of the RNA samples and setting of the reaction conditions were performed in accordance with the protocol attached to the product.

**[Table 4]**

| Gene | Forward primer | | Reverse primer | |
|---|---|---|---|---|
| | Base Sequence | SEQ ID NO | Base sequence | SEQ ID NO |
| Notch1 | CACAAGGTGTCTTCCAGATCCT | 1 | GGCATCCACATTGTTCACGG | 2 |
| Nestin | GCGTTGGAACAGAGGTTGGA | 3 | GAGCGATCTGGCTCTGTAGG | 4 |
| SOX2 | CATGGACAGTTACGCGCAC | 5 | AGCTGGTCATGGAGTTGTACTG | 6 |
| S100B | GGTGAGACAAGGAAGAGGATGT | 7 | GATGAGCTCCTTCAGTTCGGA | 8 |
| NG2 | TGCTGTTCTCACACAGAGGAAC | 9 | TTCAGCGAGAGGAGCACTTG | 10 |
| GFAP | CTTGCAGGAGTACCAGGACC | 11 | TTGAGGTGGCCTTCTGACAC | 12 |
| ACTB (control) | TGAAGGTCGGAGTCAACGATTTGGT | 13 | GAAGATGGTGATGGGATTTC | 14 |
| GAPDH (control) | ATTGCCGACAGGATGCAGA | 15 | GAGTACTTGCGCTCAGGAGGA | 16 |

Reagent: Platinum SYBR Green qPCR SuperMix-UDG (Invitrogen Corporation)
Device: StepOnePlus Real-Time PCR System (Applied Biosystems, Inc.)

The results of confirming the expression of each of neuroepithelial cell markers Notch1, Nestin, and SOX2 and a radial glial cell Nestin by quantitative PCR, and the results of confirming the expression of each of astrocyte markers GFAP and S100B and an oligodendrocyte progenitor cell marker NG2 by quantitative PCR are shown in Tables 5 and 6, respectively.

In Tables 5 and 6, the expression level of each marker gene in NHA-YA (the expression level relative to the expression level of the control gene) is shown as a relative value when the expression level of each marker gene in NHA is set to "1". Table 5 shows the expression level of each marker when the ACTB gene was used as the control gene, and Table 6 shows the expression level of each marker when the GAPDH gene was used as the control gene.

**[Table 5]**

| Type of gene | | Expression level |
|---|---|---|
| Target | Control | NHA-YA |
| Notch1 | ACTB | 0.70 |
| Nestin | ACTB | 0.33 |
| SOX2 | ACTB | 0.26 |
| GFAP | ACTB | 2.48 |
| S100B | ACTB | 5.32 |
| NG2 | ACTB | 4.06 |

**[Table 6]**

| Type of gene | | Expression level |
|---|---|---|
| Target | Control | NHA-YA |
| Notch1 | GAPDH | 0.53 |
| Nestin | GAPDH | 0.25 |
| SOX2 | GAPDH | 0.20 |
| GFAP | GAPDH | 1.89 |
| S100B | GAPDH | 4.04 |
| NG2 | GAPDH | 3.08 |

As indicated in Tables 5 and 6, in the obtained cell population (NHA-YA), higher levels of expression were confirmed for both the astrocyte markers GFAP and S100B and the oligodendrocyte progenitor cell marker NG2 compared to those in the astrocytes which were used as the raw material (NHA) .

From these results, it was found that the obtained cell population (NHA-YA) had both the characteristics of astrocytes, which are mature cells, and the characteristics of oligodendrocyte progenitor cells, which are progenitor cells, and the cells had higher proliferative ability than astrocytes. This NHA-YA can proliferate more rapidly and in greater numbers than astrocytes. Furthermore, it is found that using NHA-YA is advantageous when obtaining more exosomes in a short period of time.

### Example 3-2: Confirmation of Ectodermal Cell Markers by Quantitative PCR (2)

Expression levels of the following ectodermal cell marker genes were measured by quantitative PCR in the same manner as in Example 3-1, in the astrocyte cell group which was used as the starting material for the culturing in Example 1 and the obtained ectodermal cell population. Detection primers for each marker gene were prepared based on the sequences registered with the accession numbers in an NCBI database RefSeq, which are indicated below.
·SLC1A2 (solute carrier family 1 member 2) (including v1, v2, v3, and vX1 to vX7) (NM_004171)
·SLC1A3 (solute carrier family 1 member 3) (v1 to v5, vX1, vX2, and vX3) (NM_004172)
·OLIG2 (oligodendrocyte transcription factor 2) (NM_005806)
·PAX6 (paired box 6) (v1 to v48) (NM_000280)
·ALDH1L1 (aldehyde dehydrogenase 1 family member L1) (v1, v2, v3, vX1, vX2, and vX3) (NM_001270364)
·Musashi1 (musashi RNA binding protein 1; MSI1) (v1 and vX1 to vX10) (NM_002442)

The detection primers prepared are shown in the following Table 7. Using these primers, the expression of the ectodermal cell marker genes was examined by quantitative PCR. Preparation of the RNA samples and setting of the reaction conditions were performed in accordance with the protocol attached to the product. The measurement devices used for the quantitative PCR were SimpliAmp Thermal Cycler (Applied Biosystems, Inc.) or CFX96 Touch Real-time PCR Detection system (Bio-Rad Laboratories, Inc.) for Musashi1, and the same measurement device as that used in Example 3-1 for other marker genes.

**[Table 7]**

| Gene | Forward primer | | Reverse primer | |
|---|---|---|---|---|
| | Base sequence | SEQ ID NO | Base sequence | SEQ ID NO |
| SLC1A2 | GCCGGATAGTGCTGAAGAGG | 17 | GGCATATTGTTGGCACCTTCC | 18 |
| SLC1A3 | TGACATCTGTCGGCCTGC | 19 | GGAGGCGATCCAGGAACC | 20 |
| OLIG2 | ATAGATCGACGCGACACCAG | 21 | CTTTCGCAGGGTCCTCCTTC | 22 |
| PAX6 | CAAGCAACAACAGCAGCACA | 23 | CGGATGTCTGTCCACTCTCAC | 24 |
| ALDH1L1 | CGTGCGGAGAGTGGTAGAAG | 25 | GGCAGTACTCCATCAGCTTCA | 26 |
| Musashi1 | AATTCCGTGTAGAGCGGACC | 27 | GCAGTGAGAGGAATGGCTGT | 28 |

The results are shown in Tables 8 and 9. In Tables 8 and 9, the expression level of each marker gene in NHA-YA (the expression level relative to the expression level of the control gene) is shown as a relative value when the expression level of each marker gene in NHA is set to "1". Table 8 shows the expression level of each marker when the ACTB gene was used as the control gene, and Table 9 shows the expression level of each marker when the GAPDH gene was used as the control gene.

**[Table 8]**

| Type of gene | | Expression level |
|---|---|---|
| Target | Control | NHA-YA |
| SLC1A2 | ACTB | 12.52 |
| SLC1A3 | ACTB | 1.22 |
| OLIG2 | ACTB | 0.09 |
| PAX6 | ACTB | 0.22 |
| ALDH1L1 | ACTB | 0.25 |
| Musashi1 | ACTB | 0.83 |

**[Table 9]**

| Type of gene | | Expression level |
|---|---|---|
| Target | Control | NHA-YA |
| SLC1A2 | GAPDH | 9.49 |
| SLC1A3 | GAPDH | 0.92 |
| OLlG2 | GAPDH | 0.07 |
| PAX 6 | GAPDH | 0.17 |
| ALDH1L1 | GAPDH | 0.16 |
| Musashi1 | GAPDH | 0.72 |

### Example 4: Proteome Analysis of NHA-YA-Derived Exosomes

### <Collection of NHA-YA-Derived Exosomes>

A frozen tube in which a cell culture solution containing NHA was stored was thawed in a water bath at 37°C. Next, the lysed cells were transferred to the YA-containing culture medium so that the total volume was 10 mL and subjected to centrifugation at 180 × g for 3 minutes at 25°C, and then the medium was removed. The obtained NHA was suspended in the YA-containing culture medium, and passaged into a 6-well plate at a seeding density of 4.2 × 10³ cells/cm². While continuing the passaging twice, the culturing was performed for 11 days in the YA-containing culture medium.

Then, the YA-containing culture medium was replaced with a collection medium at a volume of a 20 mL/150 mm dish, and additional culturing was performed at 37°C for 72 hours. As the collection medium, a medium containing FBS from which exosomes were removed [3%(v/v)], instead of FBS in the YA-containing culture medium, was used. The additional culturing was performed at a seeding density of 1.16 × 10⁵ cells/150-mm dish. After the additional culturing, the culture supernatant of the additional culture was collected from the dish, transferred to a tube, subjected to centrifugation at 2,000 × g for 10 minutes at 4°C, and then filtered with a 0.22-µM filter system, whereby an exosome-containing solution was prepared.

### <Proteome Analysis of Exosomes 1>

The exosome-containing solution obtained above was subjected to a precipitation treatment with trichloroacetic acid, and then a reduction treatment was performed at 35°C for 2 hours by adding 5 mM Tris buffer solution of dithiosteitol thereto. 14 mM Tris buffer solution of iodoacetamide was added to the reduced sample, and reaction was performed at 25°C for 30 minutes under light-shielded conditions. Then, a trypsin treatment was performed at 37°C for 20 hours, so as to degrade exosomes. The obtained degradation product of exosomes was subjected to solvent displacement in a cation exchange column, and then was desalted and concentrated so as to be provided for LC-MS/MS analysis. As the HPLC device, EASY-nLC 1200 System (Thermo Fisher Scientific Inc., USA) was used, and as the column, EASY-Spray column, 15 cm × 75 µM ID, 3 µM particles, 100 Å pore size (Thermo Fisher Scientific Inc., USA) was used. A database search was performed using MASCOT server (https://www.matrixscience.com/help.html), based on the product ion measurement data of the spectrum obtained by the LC-MS/MS analysis. Next, the obtained results were compared with the following two databases, and a total of 1,798 types of protein fragments were detected in the exosome fraction and medium components combined.
·Human-derived proteins: SwissProt (number of sequences: 20,376)
·Bovine-derived proteins: UniProtKB (number of sequences: 47,043)

From a total of 1,285 marker proteins in the exosome fraction which were detected on SwissProt, those with a Quantitative Value (number of detected spectra, with correction) of 5 or more were selected, and 520 marker proteins were extracted. Characteristic proteins were selected from the 520 extracted proteins to confirm that the selected proteins were not components of the medium, and in a case where they were bovine-derived proteins, identification probability for human-derived proteins was examined, and those with a high identification probability were selected. The selection steps for the characteristic proteins are described below.

a: Selection steps for proteins characteristic of cerebrum
(a1) The 520 proteins extracted above were searched on Tissue Enrich (https://tissueenrich.gdcb.iastate.edu). Here, all of the Human Protein Atlas dataset and tissue-specific genes were searched;
(a2) 22 genes specific to cerebrum were selected;
(a3) among the 22 genes selected in (a2) above, 12 genes were selected which were not detected in the medium sample;
(a4) among the 12 gene selected in (a3) above, 8 genes were selected which were likely to be human proteins;
(a5) among the 8 genes selected in (a4) above, 5 genes were selected which were highly associated with a disease.
The proteins encoded by the 5 genes selected by the steps (a1) to (a5) above are indicated in Fig. 4.

b: Selection steps for proteins characteristic of membrane proteins, extracellular domain, and the like
(b1) Among the 520 proteins extracted above, 409 proteins were selected as proteins grouped in "membrane" by GO in Scaffold Proteome viewer;
(b2) among the 520 proteins extracted above, 443 proteins were selected as proteins grouped in "extracellular region" by GO in Scaffold Proteome viewer;
(b3) among the 520 proteins extracted above, 15 proteins were selected as proteins grouped in neither "membrane" nor "extracellular region" by GO in Scaffold Proteome viewer;
(b4) among the proteins extracted in the steps (b1), (b2), and (b3) above, 35 proteins were selected as proteins not contained in the medium sample. Of the 35 selected proteins, 14 proteins overlap with the proteins selected by the following steps (c1) to (c3).
The 35 proteins selected by the steps (b1) to (b4) above are indicated in Fig. 4.

### <Proteome Analysis of Exosomes 2>

Characteristic proteins were selected in the selection step c, which was different from the selection step a and selection step b above.
c: Selection steps for characteristic proteins
(c1) From a total of 1,562 sequences in SwissProt_Homo sapiens and UniProtKB_Bos taurus combined, 103 sequences of exosome fraction were selected which had Quantitative Values of 20 or more;
(c2) among the 103 sequences selected in (c1) above, 65 sequences in the medium samples were selected which had Quantitative Values of 10 or less;
(c3) among the 65 sequences selected in the step (c2) above, 20 sequences associated with central nervous system diseases or presumed to be involved in proliferation, development, differentiation, morphogenesis, migration, or metabolism of brain and neurons were selected from an article search site PubMed (https://pubmed.ncbi.nlm.nih.gov/). Of the 20 selected proteins, 14 proteins overlap with the proteins selected by the steps (b1) to (b4) above.
The 20 proteins selected by the steps (c1) to (c3) above are indicated in Fig. 4.

Through the above selection steps a to c, a total of 46 proteins were able to be selected (see Fig. 4).

### Example 5: miRNA Analysis of NHA-YA-Derived Exosomes

### <Collection of NHA-YA-Derived Exosomes>

A frozen tube in which a cell culture solution containing NHA was stored was thawed in a water bath at 37°C. Next, the lysed cells were transferred to the YA-containing culture medium so that the total volume was 10 mL and subjected to centrifugation at 180 × g for 3 minutes at 25°C, and then the medium was removed. The obtained NHA was suspended in the YA-containing culture medium, and passaged into a 6-well plate at a seeding density of 4.2 × 10³ cells/cm². While continuing the passaging 4 times, the culturing was performed for 47 days in the YA-containing culture medium.

Then, the YA-containing culture medium was replaced with a collection medium at a volume of a 20 mL/150 mm dish, and additional culturing was performed at 37°C for 48 hours. As the collection medium, the collection medium used in Example 4 was used. The additional culturing was performed at a seeding density of 1.16 × 10⁶ cells/150-mm dish. After the additional culturing, the culture supernatant of the additional culture was collected from the dish, transferred to a tube, subjected to centrifugation at 2,000 × g for 10 minutes at 4°C, and then filtered with a 0.22-µM filter system, whereby an exosome-containing solution was prepared.

### <Extraction of RNA from NHA-YA-Derived Exosomes>

205 mL of the exosome-containing solution obtained above was subjected to centrifugation at 250,000 × g for 70 minutes at 4°C using an ultracentrifuge Optima XE-90 (Beckman Coulter, Inc.), and then the supernatant was removed, thereby obtaining an exosome fraction. Next, RNA was extracted from the obtained exosome fraction using miRNeasy Mini Kit (217004, Qiagen). As a result, 41.8 ng of total RNA was collected from 230 µL of the exosome fraction.

### <RNA Analysis>

The RNA obtained above was subjected to quality check by Agilent 2100 Bioanalyzer, using Agilent RNA 6000 pico kit (Agilent Technologies, Inc.) and Agilent small RNA kit (Agilent Technologies, Inc.). After the quality check, an RNA library was prepared from the total RNA obtained above, using directional (stranded) RNA library preparation kit and NEBNext Ultra II Directional RNA Library Prep Kit for Illumina. mRNA-Seq analysis was performed using the prepared RNA library, and 6,538 mRNAs were detected.

In a similar manner, a miRNA library was prepared from the total RNA obtained above, using a miRNA library preparation kit, QIAseq miRNA Library Kit (Qiagen), and QIAseq miRNA NSG 96 Index IL (Qiagen). miRNA-Seq analysis was performed using the prepared miRNA library, and 370 miRNAs were detected.

Quality check for the sequence library was performed by Agilent 2100 Bioanalyzer, using High Sensitivity DNA kit (Agilent Technologies, Inc.).

NGS was performed using NextSeq 500, Illumina, Inc. (single end, 75 bp, average number of reads: approximately 10 million reads).

After sequencing, the data was subjected to quality assessment for reads (FastQC) and then aligned (mapped) with the reference genome (Human hg38) on GeneGlobe: Data Analysis Center (QIAGEN), the expression levels were subjected to Trimmed mean of M values (TMM) normalization, and an Excel format file containing annotation information of each miRNA and classification summary of Small RNA composition was created (analysis tool: Strand NGS v4.0, R v3.6.2; annotation information: based on miRBase Release 21). Next, as data analysis, genes with variable expression were extracted, and then GO analysis and Pathway analysis were conducted from target gene prediction.

### <Selection of Functional Marker miRNA>

### 1. miRNA as Parkinson's Disease Marker

With reference to the following literature, 42 miRNAs were selected, of which the expression is decreased in human brain tissue with Parkinson's disease. By administering exosomes containing the miRNAs into the cells of a Parkinson's disease patient, the functions of the miRNAs are exogenously complemented in the Parkinson's disease patient, which is expected to be effective in the treatment of Parkinson's disease.
Literature: MicroRNAs in Parkinson's disease and emerging therapeutic targets. Neural Regeneration Research, 12(12), pp.1945-1959 (2017)

Next, among these miRNAs, 16 miRNAs were selected, which were detected as miRNAs of the NHA-YA-derived exosomes. The 16 selected miRNAs are shown in Fig. 5.

### 2. miRNA as Alzheimer's Disease Marker

Using IMOTA (https://ccb-web.cs.uni-saarland.de/imota/), miRNAs were selected which were related to proteins associated with the pathology of Alzheimer's disease, particularly, the following proteins known to be associated with a plurality of proteins that contribute to the pharmacological action of a therapeutic agent for Alzheimer's disease.

### 2-1. miRNA as Alzheimer's Disease Marker (1)

Using IMOTA, 70 miRNAs were selected which were associated with amyloid precursor protein (APP) in cerebral cortex. APP is a major component of senile plaque, that is, an amyloid β protein deposit, which is known as one of the causes of Alzheimer's disease.

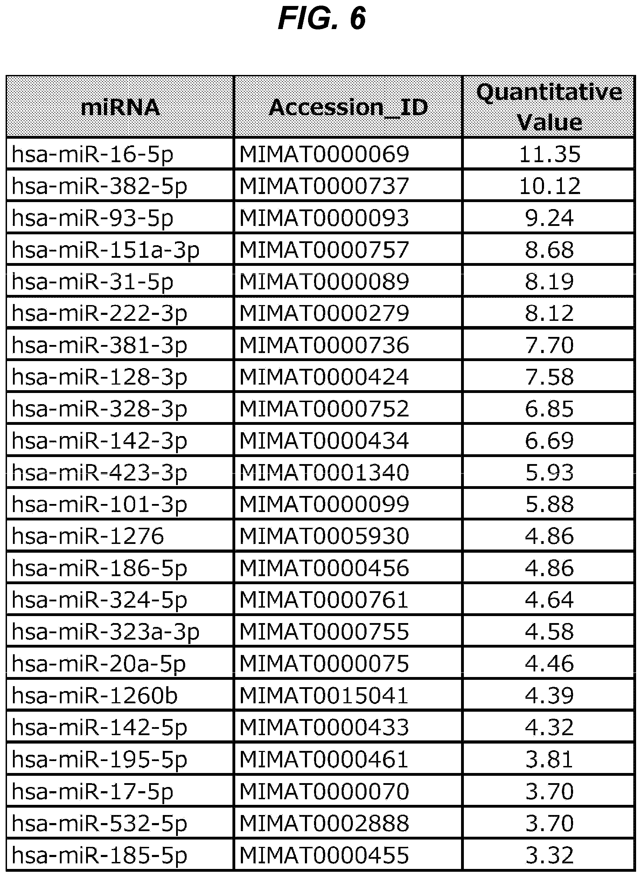

Next, among these miRNAs, 23 miRNAs were selected, which were detected as miRNAs of the NHA-YA-derived exosomes. The 23 selected miRNAs are shown in Fig. 6.

### 2-2. miRNA as Alzheimer's Disease Marker (2)

Using IMOTA, 42 miRNAs were selected which were associated with the β-site APP cleaving enzyme (BACE1) in cerebral cortex. At the onset of Alzheimer's disease, BACE1 cleaves the N-terminal moiety of APP, thus producing abnormal amyloid β protein.

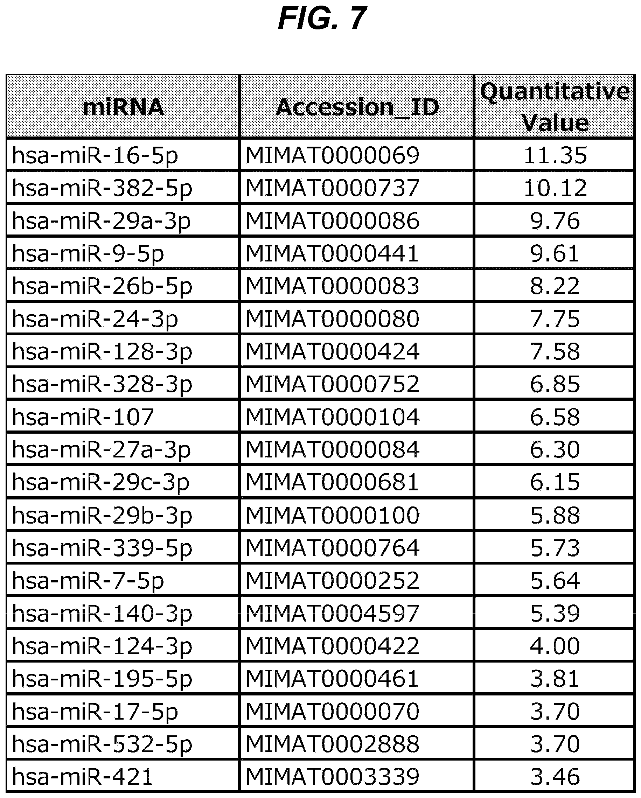

Next, among these miRNAs, 20 miRNAs were selected, which were detected as miRNAs of the NHA-YA-derived exosomes. The 20 selected miRNAs are shown in Fig. 7.

### 2-3. miRNA as Alzheimer's Disease Marker (3)

Using IMOTA (https://ccb-web.cs.uni-saarland.de/imota/), 66 miRNAs were selected which were associated with the N-methyl-D-aspartate receptor (NMDA receptor) in cerebral cortex. At the onset of Alzheimer's disease, abnormal proteins accumulate in the brain, which causes excessive release of a substance that excites the nerve. Due to this excitatory substance, the NMDA receptor is excessively activated, whereby neurotransmission and memory are impaired.

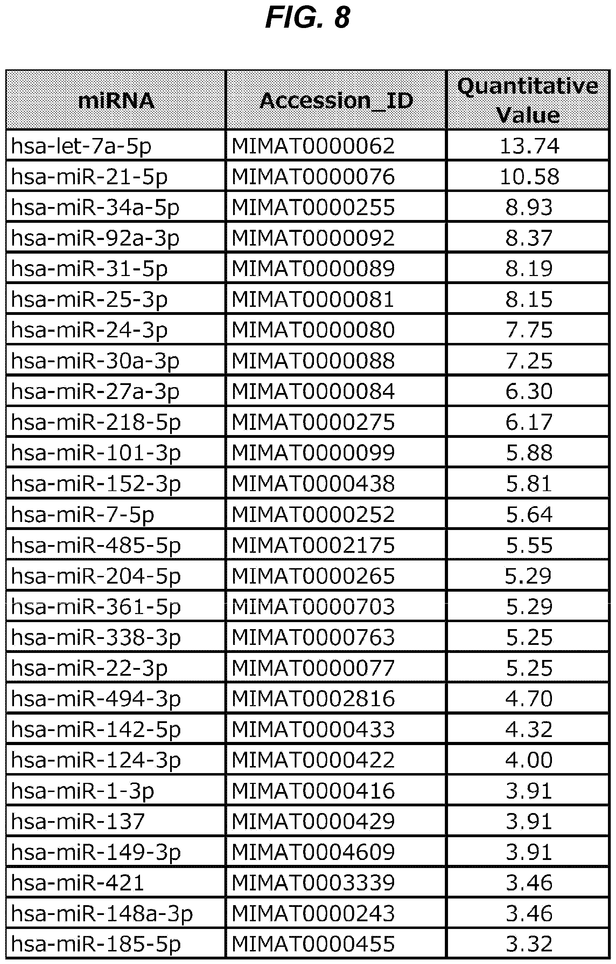

Next, among these miRNAs, 27 miRNAs were selected, which were detected as miRNAs of the NHA-YA-derived exosomes. The 27 selected miRNAs are shown in Fig. 8.

### 2-4. miRNA as Alzheimer's Disease Marker (4)

Using IMOTA (https://ccb-web.cs.uni-saarland.de/imota/), 58 miRNAs were selected which were associated with glucogen synthase kinase-3β (GSK-3β) in cerebral cortex. GSK-3β is an enzyme that phosphorylates various proteins and plays a variety of roles in maintaining cell life and regulating physiological functions through the regulation of multiple pathways. In Alzheimer's disease, GSK-3β promotes amyloid β protein deposition in the brain and tau protein accumulation in neurons. As a result, apoptosis of neurons is induced.

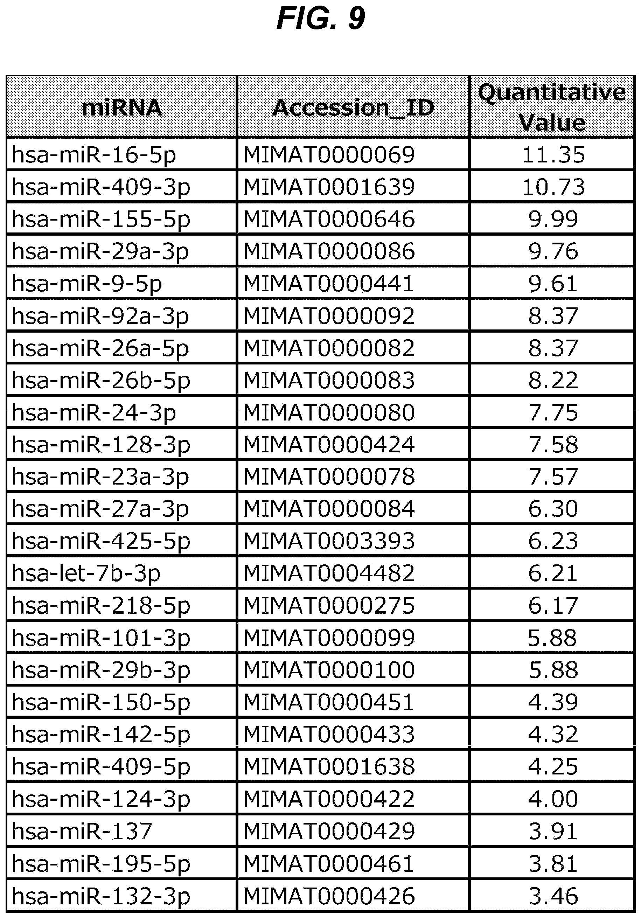

Next, among these miRNAs, 24 miRNAs were selected, which were detected as miRNAs of the NHA-YA-derived exosomes. The 24 selected miRNAs are shown in Fig. 9.

### 3. miRNA as Depression Marker

With reference to the following literature, 24 miRNAs were selected, of which the expression is decreased in the plasma of an individual with depression. By administering exosomes containing the miRNAs into the cells of a depression patient, the functions of the miRNAs are exogenously complemented in the depression patient, which is expected to be effective in the treatment of depression.

Literature: MicroRNAs expressed in depression and their associated pathways: A systematic review and a bioinformatics analysis (Journal of Chemical Neuroanatomy 100 (2019) 101650)

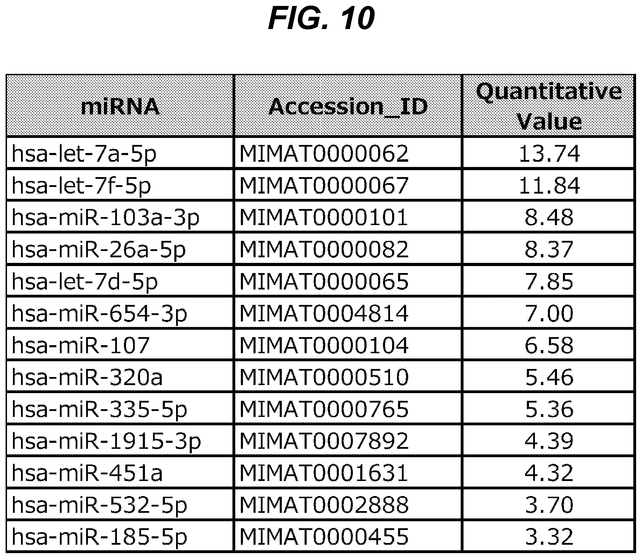

Next, among these miRNAs, 13 miRNAs were selected, which were detected as miRNAs of the NHA-YA-derived exosomes. The 13 selected miRNAs are shown in Fig. 10.

### Example 6: Neurite Outgrowth Suppression Test of PC-12 Cells Using NHA-YA-Derived Exosomes

As a functionality evaluation test using NHA-YA-derived exosomes, a neurite outgrowth suppression test was conducted on a rat adrenal pheochromocytoma-derived cell line, PC-12 cells (RCB0009, Cell Bank, RIKEN BioResource Research Center). As a control, exosomes derived from NHA cells used in Example 1 were used.

### <Reagents and Products for Culturing>

The following reagents and products for culturing were used.
- Growth medium:
   Composition: basal medium, 10% (w/v) FBS, 10% (w/v) HS, antibiotic,
   basal medium: DMEM, high glucose, pyruvate (11995-073; Gibco),
   FBS: Fetal Bovine Serum, qualified, Brazil (10270-106; Gibco),
   HS: Horse Serum, heat inactivated, New Zealand origin (26050-088; Gibco),
   antibiotic: Antibiotic-Antimycotic (100X) (15240-062; Gibco)
- Cell detachment agent 1: Accutase (AT104, Innovative Cell Technologies, Inc.)
- Cell detachment agent 2: TrypLE Express Enzyme (1X), no phenol red (12604-013, Gibco),
- Cell buffer: phosphate buffered saline (Dulbecco's formula; without calcium and magnesium) (BNDSBN200, KAC Co., Ltd.)
- Assay medium:
   Composition: basal medium, additive, antibiotic,
   basal medium: Advanced DMEM (12491-015, Gibco),
   additive: GlutaMAX Supplement (100X) (35050-061, Gibco),
   antibiotic: Antibiotic-Antimycotic (100X) (15240-062, Gibco),
- Positive control reagent: rat-derived nerve growth factor (NGF)-β (N2513-.1MG, Sigma-Aldrich, Inc.),
- Culture medium: AGM Astrocyte Growth Medium Bullet Kit (CC-3186, Lonza Group AG) (composition of AGM: basasl medium, FBS (3% (v/v)), L-glutamine, ascorbic acid, hEGF, insulin, and antibiotic) (the same culture medium used in Example 1)
- Inhibitor Y: CultureSure (registered trademark) Y-27632 (034-24024, FUJIFILM Wako Pure Chemical Corporation), final concentration:10 µM (the same inhibitor used in Example 1)
- Inhibitor A: CultureSure (registered trademark) A-83-01 (035-24113, FUJIFILM Wako Pure Chemical Corporation), final concentration:0.5 µM (the same inhibitor used in Example 1)
- Exosome-depleted bovine serum: Exosome-Depleted Fetal Bovine Serum Qualified One shot (A2720803, Gibco)
- 0.22-µM filter system: Stericup Quick Release-GP Sterile Vacuum Filtration System (S2GPU02RE, Merck Millipore)
- Culture supernatant collection medium: a medium obtained by adding 3% (w/v) exosome-depleted bovine serum (A2720803; Gibco) instead of bovine serum (FBS), which is a component of AGM Astrocyte Growth Medium Bullet Kit (CC-3186, Lonza Group AG)
- 96-Well plate: collagen I coat 96-well plate (4860-010, IWAKI)
- Cell culturing flask T-75 (430641, Corning Incorporated),
- Cell culturing dish 100 mm: 150466, Thermo Fisher Scientific Inc. (the same dish used in Example 1)
- Cell culturing dish 150 mm: 150468, Thermo Fisher Scientific Inc. (the same dish used in Example 1)

### <Preparation of Samples for Evaluation>

The following samples for evaluation were prepared in accordance with the procedures below, and the samples were used in the neurite outgrowth suppression test.
(1) Control medium (CM): medium obtained by subjecting the culture supernatant collection medium to ultracentrifugation,
(2) NHA AGM P6: sample obtained by subjecting culture supernatant of NHA, which had been passaged 6 times, to ultracentrifugation,
(3) NHA-YA P6: sample obtained by subjecting culture supernatant of NHA-YA, which had been passaged 6 times, to ultracentrifugation.
(2) and (3) above were prepared by adding exosomes to the assay medium so that the final concentrations of the exosomes were 10 µg/mL. In addition, the ultracentrifugation used when preparing the samples for evaluation (1) to (3) above was performed using an ultracentrifuge (main body: Optima XE-90, rotor: SW41 Ti; Beckman Coulter, Inc.) under the conditions of 250,000 × g, 70 minutes, and 4°C.

### <Collection and Identification of Exosomes in Culture Supernatant>

Using the following materials, reagents, and products for culturing, the culture supernatant obtained when the human astrocyte cells were cultured using a YA-containing culture medium or the inhibitor-free medium was collected.

### 1. Materials

As NHA, the same materials as those used in Example 1 were used.

### 2. Collection of Supernatant of Human Astrocyte Cell Culture

The culture supernatant was collected in accordance with the following procedure. A frozen tube of the human astrocyte cells (NHA) was thawed in a water bath at 37°C. Next, the lysed cells were transferred to the culture medium so that the total volume was 10 mL and subjected to centrifugation at 180 × g for 3 minutes at room temperature, and then the medium was removed. The cells were suspended in the culture medium and seeded in a T-75 culturing flask (Corning Incorporated) at a seeding density of 1.25 × 10⁴ cells/cm². Next, culturing was performed at 37°C for about 12 hours, and, after adhering the cells, the medium was replaced with any of the culture mediums of "inhibitor Y + inhibitor A-added group (hereinafter, NHA-YA group)" or "inhibitor-free group (NHA group)" (the concentration of each inhibitor is as described above), and the culturing was continued.

The medium was replaced every other day or every two days with fresh culture medium containing each inhibitor (or not containing any inhibitor). After 7 days of culturing, the cells were detached using the cell detachment agent 1 (passage number: 1), suspended in each of the inhibitor-containing culture medium, that is, the YA-containing culture medium, and the inhibitor-free culture medium, and passaged into a 150-mm cell culturing dish at a seeding density of 3.3 × 10⁴ cells/cm². After 9 days of culturing, by the same method, the culture was repeatedly passaged at an interval of 5 days in a 150-mm cell culturing dish at a seeding density of 3.3 to 6.6 × 10⁴ cells/cm², and the passaging was performed a total of 5 times over 31 days in total. As in Example 1, the cell group cultured in the YA-containing culture medium is referred to as the NHA-YA group, and the cell group cultured in the inhibitor-free culture medium is referred to as the normal group.

At the sixth passaging, the NHA-YA group was seeded in five 150-mm culturing dishes at a seeding density of 3.3 × 10⁴ cells/cm², and the normal group was seeded in five 150-mm culturing dishes at a seeding density of 2.3 × 10⁴ cells/cm². The total number of seeded cells was 5.0 × 10⁶ cells in the NHA-YA group, and 3.5 × 10⁶ cells in the normal group.

Culturing was performed for 4 days after the seeding, and it was confirmed that, on Day 35 after induction (after the contact with the inhibitor), 40 to 60% of confluency was reached. The culture medium was replaced with the culture supernatant collection medium at a volume of a 20 mL/150 mm dish, and additional culturing was started. 48 hours after the start of the additional culturing, the culture supernatant of the additional culture was collected from the dish, and then the collected culture supernatant was filtered with a 0.22-µM filter system to obtain an exosome-containing culture supernatant from which cell debris had been removed. The obtained filtered exosome-containing culture supernatant was stored at 4°C. Next, a fresh culture supernatant collection medium was added to the cells remaining in the dish, culturing was performed for 48 hours, and the filtered exosome-containing culture supernatant was obtained by the same method. The same operation was performed a total of 3 times, and about 300 mL of the filtered exosome-containing culture supernatant was collected. On Day 41 after the induction, the culture supernatant was collected, the cells remaining in the dish was detached using the cell detachment agent 1, and the number of cells was counted. As a result, the total number of obtained cells was 4.42 × 10⁷ cells in the NHA-YA group and 1.51 × 10⁷ cells in the NHA group, and the residual ratio relative to the total number of seeded cells was 94.1% in the NHA-YA group and 84.0% in the normal group.

As the control medium (CM) for the evaluation of the exosome function, the culture supernatant collection medium was added to a 150-mm cell culturing dish in which cells were not seeded at a volume of a 20 mL/150 mm dish, and the culture supernatant was collected under exactly the same conditions as those for culturing the cells.

### 3. Concentration of Exosomes in Culture Supernatant

180 mL of the culture supernatants of the NHA-YA group, the NHA group, and CM collected as described above were transferred to tubes, and centrifugation was performed at 250,000 × g for 70 minutes at 4°C using the above ultracentrifuge. After removing the supernatants, phosphate buffered saline was added to the obtained precipitates in order to wash the precipitates, and then centrifugation was performed at 250,000 × g for 70 minutes at 4°C. The supernatants were removed, thus obtaining washed precipitates. The washed precipitates were resuspended in phosphate buffered saline to about 1/1,000 the volumes of the culture supernatants.

### 4. Measurement of Number of Particles and Amount of Protein in Suspension

The culture supernatant-derived suspension concentrated by the method described above was diluted 100-fold with phosphate buffered saline that had been filtered with a 0.22-µM filter before use, and then the number of particles in the diluted solution was measured using ZetaView X20 (Particle Metrix GmbH). The number of particles obtained by the measurement was multiplied by the dilution ratio, that is, 100, to calculate the total number of particles in the suspension. The number of particles in the assay medium was measured after diluting the medium 100-fold with phosphate buffered saline, and the number of particles in the culture supernatant was calculated by subtracting the obtained number of particles in the assay medium from the measurement value for each culture supernatant. In addition, absorbance was measured at 280 nm using NanoDrop (Thermo Fisher Scientific Inc.), so as to measure the protein concentration in the suspension. The total amount of protein in the suspension was calculated by multiplying the protein concentration obtained by the measurement by the fluid volume of the suspension. The measurement results are shown in Table 10. In Table 10, "aE + b" in the column of "Total number of particles" indicates a × 10^{b}. As shown in Table 10, it is found that the total amount of protein was the greatest in the NHA-YA group, and the number of particles was also significantly greater in the NHA-YA group than in the control medium (CM) .

**[Table 10]**

| Sample for evaluation | Protein concentration (µg/mL) | Fluid volume (µL) | Total amount of protein (µg) | Total number of particles |
|---|---|---|---|---|
| (1) Control medium (CM) | 26 | 180 | 4.68 | 2.89E + 08 |
| (2) NHA AGM P6 | 123 | 180 | 22.14 | 5.00E + 10 |
| (3) NHA-YA P6 | 292 | 180 | 52.56 | 4.63E + 10 |

### <Evaluation Test Protocol>

A frozen tube of the PC-12 cells was thawed in a water bath at 37°C. Next, the lysed cells were transferred to the growth medium so that the total volume was 10 mL and subjected to centrifugation at 180 × g for 5 minutes at room temperature, and then the medium was removed. Then, the cells were suspended in the growth medium and seeded in a cell culturing dish 100 mm at a seeding density of about 5,300 cells/cm². Culturing was performed at 37°C while replacing the medium every 2 or 3 days, and the cells were allowed to proliferate while continuing passaging. The obtained PC-12 cells were detached using the cell detachment agent 2, washed with the assay medium, and then resuspended in the assay medium at a density of 3.96 × 10⁴ cells/mL. The suspended cells were seeded in a 96-well plate at a seeding density of 1.98 × 10³ cells /50 µL medium per well, that is, 6,000 cells/cm², and the cells were adhered for about 12 hours. Then, the medium was replaced with each of the samples for evaluation. Next, culturing was performed at 37°C, the conditions of the PC-12 cells were observed with a microscope (BZ-X710, KEYENCE CORPORATION) on Days 3, 4, and 5 after the start of the culturing, and the observation images were obtained. The length of the nerve-like projection and the number of cells in the images were measured using the Multi-point tool (number of cells) and Straight tool (elongated projection length) of image analysis software Fiji (ImageJ2 ver. 2.3.0). Microsoft-Excel was used for statistical processing.

As a result, in the PC-12 cell line, which are sympathetic nerve cells, compared to (1) above, no significant neurite outgrowth was observed in (2) and (3) that contained exosomes, indicating that neurite outgrowth was suppressed.

Therefore, it is suggested that the exosomes of the present disclosure do not have the function of causing neurite outgrowth in PC-12 cells, but rather have the function of suppressing neurite outgrowth. Thus, the exosomes of the present disclosure can suppress the sympathetic nervous system.

## Claims

1. A method for producing highly proliferating cells, the method comprising:
(i) preparing ectodermal cells which serve as a raw material;
(ii) culturing the ectodermal cells which serve as the raw material in a medium containing a small-molecule signaling pathway inhibitor by bringing the inhibitor into contact with the ectodermal cells; and
(iii) after the contact, performing additional culturing in a medium containing the inhibitor to obtain a culture containing highly proliferating cells that have higher cell proliferative ability than the ectodermal cells which serve as the raw material.

2. The production method according to claim 1, wherein the highly proliferating cells have proliferative ability that allows the number of the highly proliferating cells after more than 28 days of a culturing period during which the cells are brought into contact with the inhibitor to be greater than 1.0 times the number of ectodermal cells which are cultured under the same culturing conditions, except that the cells are not brought into contact with the inhibitor, for the same culturing period.

3. The production method according to claim 1 or 2, wherein the ectodermal cells which serve as the raw material comprise cells of the central nervous system.

4. The production method according to any one of claims 1 to 3, wherein the ectodermal cells which serve as the raw material comprise astrocytes.

5. The production method according to any one of claims 1 to 4, wherein the inhibitor comprises at least one compound selected from the group consisting of a TGFβ receptor inhibitor and a ROCK inhibitor.

6. The production method according to claim 5, wherein the concentration of the TGFβ receptor inhibitor is in a range of 0.001 µM to 100 µM.

7. The production method according to claim 5 or 6, wherein the concentration of the ROCK inhibitor is in a range of 0.001 µM to 100 µM.

8. The production method according to any one of claims 1 to 7, wherein the highly proliferating cells express at least one gene specific to mature cells at the same level as or at a higher level than the ectodermal cells which serve as the raw material, and express at least one gene specific to progenitor cells at the same level as or at a higher level than the ectodermal cells which serve as the raw material.

9. The production method according to any one of claims 1 to 8, wherein the highly proliferating cells are negative for at least one selected from the group consisting of Musashi1, Notch1, Nestin, and SOX2.

10. Highly proliferating cells having characteristics of ectodermal cells and proliferative ability that allows the number of the highly proliferating cells after more than 28 days of a culturing period during which the cells are brought into contact with a small-molecule signaling pathway inhibitor to be greater than 1.0 times the number of ectodermal cells which are cultured under the same culturing conditions, except that the cells are not brought into contact with the inhibitor, for the same culturing period.

11. The cells according to claim 10, wherein the highly proliferating cells comprise cells negative for at least one selected from the group consisting of Musashi1, Notch1, Nestin, and SOX2.

12. The cells according to claim 10 or 11, wherein the NG2 expression level is higher than the NG2 expression level in the ectodermal cells that are not brought into contact with the inhibitor.

13. A method for producing a cell-secreted product, the method comprising:
obtaining a culture containing a cell-secreted product secreted from the highly proliferating cells obtaining by the production method according to any one of claims 1 to 9 or the highly proliferating cells according to any one of claims 10 to 12; and
separating the cell-secreted product from the culture.

14. The method according to claim 13, wherein the cell-secreted product is an exosome.

15. A method for producing ectodermal progenitor cells, the method comprising:
(i) preparing ectodermal cells which serve as a raw material;
(ii) culturing, for a period of more than 28 days, the ectodermal cells which serve as the raw material in a medium containing a small-molecule signaling pathway inhibitor by bringing the inhibitor into contact with the ectodermal cells; and
(iii) after the contact, performing additional culturing in a medium containing the inhibitor to obtain a culture containing highly proliferating cells that have higher cell proliferative ability than the ectodermal cells which serve as the raw material,
wherein the ectodermal progenitor cells are the highly proliferating cells.

16. The production method according to claim 15, further comprising:
isolating the highly proliferating cells from the culture.

17. A cell-secreted product comprising:
a protein and/or a miRNA,
wherein the protein comprises a combination of vinculin (P18206), integrin β-1, CD29 (P05556), pyruvate kinase M1/2 (P14618), and ephrin type-A receptor 2 (P29317), and
wherein the miRNA comprises a combination of hsa-miR-382-5p (MIMAT0000737), hsa-miR-155-5p (MIMAT0000646), hsa-miR-379-5p (MIMAT0000733), hsa-miR-16-5p (MIMAT0000069), hsa-miR-382-5p (MIMAT0000737), hsa-miR-16-5p (MIMAT0000069), hsa-miR-382-5p (MIMAT0000737), hsa-miR-21-5p (MIMAT0000076), hsa-let-7a-5p (MIMAT0000062), hsa-miR-16-5p (MIMAT0000069), hsa-miR-409-3p (MIMAT0001639), hsa-let-7a-5p (MIMAT0000062), and hsa-let-7f-5p (MIMAT0000067).

18. The cell-secreted product according to claim 17, which is an exosome.

19. A pharmaceutical composition for use in prevention or treatment of a disorder associated with peripheral nerve cells or central nerve cells, the pharmaceutical composition comprising any one of:
a cell-secreted product secreted from the highly proliferating cells obtained by the production method according to claim 1 or 2 or from the highly proliferating cells according to any one of claims 10 to 12, and
the cell-secreted product according to claim 17 or 18.

20. A method for inhibiting sympathetic nervous system, the method comprising:
bringing neurons into contact with any one of:
a cell-secreted product secreted from the highly proliferating cells obtained by the production method according to claim 1 or 2 or from the highly proliferating cells according to any one of claims 10 to 12, and
the cell-secreted product according to claim 17 or 18.
